# EUROPEAN PATENT APPLICATION

(11) **EP 1 721 604 A1**
(43) Date of publication of application: **15.11.2006**
(21) Application number: 05719925.9
(22) Date of filing: 03.03.2005
(51) Int. Cl.: A61K 9/48, A61K 9/58, A61K 31/4439, A61K 45/00, A61K 47/34, A61K 47/42, A61P 1/04, C07D 401/12

(54) **STABLE CAPSULE PREPARATION**

(30) Priority: 04.03.2004 JP 2004060613
(71) Applicant: Takeda Pharmaceutical Company Limited, Osaka 540-08645 (JP)
(72) Inventor: NAGAHARA, N.; c/o Takeda Pharmaceutical Co. Ltd., Osaka-shi, Osaka 5328686 (JP); ITO, Hiroki, Osaka 5650872 (JP); NONOMURA, M.; c/o Takeda Pharmaceutical Co. Ltd., Osaka-shi, Osaka 5328686 (JP)
(74) Representative: Wright, Robert Gordon McRae
(86) International application number: PCT/JP2005/003621
(87) International publication number: WO 2005/084649

(57) **Abstract**

A capsule in which an unstable active ingredient has been stabilized is obtained by lowering the moisture content of a solid preparation (granules, subtilis, tablets, etc.) containing a chemical unstable to moisture such as an imidazole PPI compound by drying or the like, and then filling in a capsule comprising a water-soluble polysaccharide such as pullulan as the main component or a PEG-containing gelatin capsule. For the further stabilization, the capsule per se may be dried. The capsule preparation thus obtained is a stable capsule preparation with the use of a capsule being stable at a low moisture content which contains a chemical unstable to moisture such as an imidazole compound.

## Description

### Technical Field

The present invention relates to a stable capsule preparation using a capsule being stable in a low moisture state which comprises a medicine unstable to moisture, for example, PPI of an imidazole type compound.

### Background Art

A benzimidazole type compound such as lansoprazole, omeprazole, rabeprazole, or the like has a proton pump inhibitory activity (hereinafter sometimes referred to as a PPI activity) and exerts a gastric secretion-suppressing activity or a gastric mucosa-protecting activity by binding to the SH group of a (H⁺+K⁺)-ATPase having a role as a proton pump to inhibit its enzymatic activity. These proton pump inhibitors (hereinafter referred to as "PPI") have been widely used as, for example, a therapeutic agent for peptic ulcer.

However, these compounds are extremely unstable to an acid and water and are remarkable in discoloration, degradation and the like. With respect to stability to an acid, improvement in the stability by using an enteric coating or blending a nontoxic base has been already known. Particularly, a capsule preparation containing enteric granules and enteric fine granules with an enteric coating has been known (Patent Document 1).

Further, for improvement in the stability to moisture, it is most effective to lower the moisture content of a preparation per se. However, in the case of a capsule preparation which is common as a preparation containing a benzimidazole type compound, there is such a problem that a generally used hard gelatin capsule has weak mechanical strength in a low moisture state and is apt to be broken.

On the other hand, a capsule comprising a cellulose derivative as a main component (such as a hydroxypropylmethyl cellulose capsule (hereinafter simply abbreviated as an HPMC capsule)) has mechanical strength even in a low moisture state, and a HPMC capsule preparation containing a PPI or the like as an active ingredient has been known (Patent Document 2). However, such a HPMC capsule preparation has a problem that its solubility is lower than that of a hard gelatin capsule at low pH. In order to achieve a quick effect of a medicine, it is desirable that a capsule is quickly dissolved in the stomach having low pH. Particularly, in the case of a capsule preparation containing enteric granules or enteric fine granules widely used for a benzimidazole type compound, it is necessary that the capsule is dissolved in the stomach, the granules are released from the capsule and eliminated from the stomach, and then the granules reach the intestines, followed by dissolution of the medicine from the granules and absorption of the medicine for achieving the effect of the medicine.

A preparation containing a medicine unstable to moisture, typically, a benzimidazole type compound, is desired to have a low moisture content for improvement in stability, and thus a capsule preparation filled with the medicine preferably maintains mechanical strength even at low moisture and is quickly dissolved independently of pH.
Patent Document 1: WO 03/32953 A
Patent Document 2: WO 02/39980 A

### Disclosure of the Invention

Problem to be solved by the Invention

An object of the present invention is to provide a stabilized capsule preparation comprising a pharmaceutically active ingredient unstable to moisture such as an imidazole type PPI compound.

### Means for solving the Problem

That is, the present invention provides:
(1) A capsule preparation, which comprises a medicine unstable to moisture, is stable in a low moisture state and has pH-independent disintegration properties;
(2) The capsule preparation according to the above (1), which is stable in a low moisture state which is less or equal to relative humidity of about 35%;
(3) The capsule preparation according to the above (1), wherein the main component of the capsule is a gelatin containing polyethylene glycol;
(4) The capsule preparation according to the above (1), wherein the main component of the capsule is a water-soluble polysaccharide;
(5) The capsule preparation according to the above (1), wherein the main component of the capsule is pullulan;
(6) The capsule preparation according to the above (1), which combines a capsule shell comprising gelatin containing polyethylene glycol as the main component and a capsule shell comprising pullulan as the main component;
(7) The capsule preparation according to the above (1), wherein the medicine unstable to moisture is a proton pump inhibitor (PPI);
(8) The capsule preparation according to the above (7), wherein the PPI is an imidazole type compound represented by the formula (I'): 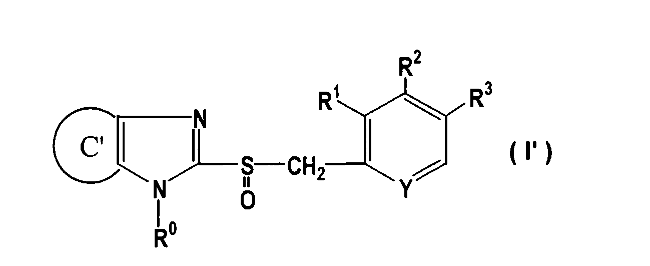 wherein the ring C' is an optionally substituted benzene ring or an optionally substituted aromatic mono-heterocyclic ring, R° is a hydrogen atom, an optionally substituted aralkyl group, an acyl group or an acyloxy group, each of R¹, R² and R³ which may be the same or different, and is a hydrogen atom, an optionally substituted alkyl group, an optionally substituted alkoxy group, or an optionally substituted amino group, and Y is a nitrogen atom or CH, or an optically active isomer thereof or a salt thereof;
(9) The capsule preparation according to the above (8), wherein C' is an optionally substituted benzene ring;
(10) The capsule preparation according to the above (7), wherein the PPI is lansoprazole, omeprazole, rabeprazole, pantoprazole, tenatoprazole, or an optically active isomer thereof or a salt thereof;
(11) The capsule preparation according to the above (7), wherein the PPI is lansoprazole;
(12) The capsule preparation according to the above (7), wherein the PPI is an optically active isomer (R-isomer) of lansoprazole;
(13) The capsule preparation according to the above (1), wherein the medicine unstable to moisture is a prodrug of PPI;
(14) The capsule preparation according to the above (1), wherein the content in the capsule is a powdered medicine;
(15) The capsule preparation according to the above (1), wherein the content in the capsule is fine granules optionally coated, granules optionally coated and/or tablets optionally coated;
(16) The capsule preparation according to the above (15), which contains at least two solid preparations selected from fine granules, granules and tablets in combination;
(17) The capsule preparation according to the above (16), wherein the combined solid preparations have different medicine release properties;
(18) The capsule preparation according to the above (16), wherein at least one of the combined solid preparations has a coating layer;
(19) The capsule preparation according to the above (18), wherein the coating layer is an enteric coating layer;
(20) The capsule preparation according to the above (18), wherein the coating layer contains a controlled-release coating layer;
(21) The capsule preparation according to the above (20), wherein the controlled-release coating layer is a pH-dependent soluble controlled-release coating film containing a polymer soluble within a range of pH 6.0 to pH 7.5;
(22) The capsule preparation according to the above (21), wherein the controlled-release coating layer is a diffusion-control type controlled-release film;
(23) The capsule preparation according to the above (21), wherein the controlled-release coating layer is a time release type controlled-release coating film; and
(24) The capsule preparation according to the above (16), which contains fine granules, granules or tablets having an enteric coating layer in combination with fine granules, granules or tablets having a controlled-release coating layer.

### Effects of the Invention

In the capsule preparation using a capsule comprising a water-soluble polysaccharide as the main component (e.g. a pullulan capsule) or a PEG-containing gelatin capsule of the present invention, the capsule per se is excellent in mechanical strength in a low moisture state and hardly broken. Therefore, the capsule is stable even when the capsule is filled with a solid preparation (e.g. a powdered medicine, fine granules, granules or tablets) containing a medicine unstable to moisture such as an imidazole type compound having a PPI activity and its moisture content is lowered for improvement in stability of the medicine, and thus the entire preparation including the capsule, not only the medicine, is stabilized. Further, since the PEG-containing gelatin capsule, the pullulan capsule, etc. used in the present invention are excellent in solubility at low pH, it will be quickly dissolved in the stomach and thus the medicinal effect will be quickly achieved.

### Best Mode for Carrying Out the Invention

The capsule preparation of the present invention is a capsule preparation in which an unstable active ingredient has been stabilized by lowering the moisture content of a medicine unstable to moisture, for example, an imidazole type compound which has a PPI activity, is extremely unstable to moisture and is represented by the above formula (I'), by drying or the like, and then filling it in a capsule comprising a water-soluble polysaccharide as the main component, a PEG-containing gelatin capsule or the like. For further stabilization, the capsule per se may be dried.

The capsule comprising a water-soluble polysaccharide as the main component (e.g. a pullulan capsule) or a gelatin capsule containing polyethylene glycol (PEG) (hereinafter simply abbreviated to a PEG-containing gelatin capsule) to be used is that excellent in mechanical strength in a low moisture state as compared with a conventional gelatin hard capsule, is hardly broken, and has pH-independent disintegration properties. Further, since the capsule is excellent in solubility at low pH as compared with a capsule comprising as the main component a cellulose derivative (e.g. a HPMC capsule) having similarly excellent mechanical strength, quick dissolution of the capsule in the stomach as well as quick onset of the medicinal effect can be expected. Thus, not only the medicine, but also the entire preparation including the capsule can be stabilized by using the capsule comprising a water-soluble polysaccharide as the main component (e.g. a capsule containing pullulan as the main component, hereinafter sometimes referred to simply as a pullulan capsule), the capsule comprising PEG-containing gelatin as the main component (hereinafter sometimes referred to simply as a PEG-containing gelatin capsule), or the like as a capsule to be filled with a solid preparation (such as a powdered medicine, fine granules, granules or tablets) containing a medicine unstable to moisture such as an imidazole type compound having a PPI activity and requiring lowering a moisture content for improvement in stability.

The capsule comprising a water-soluble polysaccharide as the main component to be used in the present invention is not limited as far as it is a capsule comprising a water-soluble polysaccharide as the main component being stable in a low moisture state, and having pH-independent disintegration properties.

The "pH-independent disintegration properties" mean such properties that the disintegration properties of a capsule shell at low pH (e.g. pH 1.2) assuming the conditions in the stomach are substantially the same as those at a neutral region (e.g. pH 6 to 7) assuming the conditions in the small intestine.

Here, the "disintegration properties" mean that the capsule shape cannot be maintained, for example, by dissolving the capsule shell in a test liquid, and the content in the capsule is released. For example, the properties can be evaluated by measuring concentration of a medicine in the content released from the capsule or by visually observing the content release time from the capsule. However, the evaluation is not limited thereto.

The disintegration properties being "substantially the same" used herein mean that a release starting time of the content from the capsule, and thereafter the release behavior of a medicine are the same, or that the content release starting time from the capsule and finish time by visual observation are the same.

The medicine dissolution behavior may be determined by measuring the change of a dissolution rate with time.

Specifically, the disintegration properties can be evaluated, for example, in accordance with Japanese Pharmacopoeia, Disintegration Test (Fourteenth Edition). Otherwise, they can be evaluated in accordance with Japanese Pharmacopoeia, Dissolution Test, Method 1 or Method 2 (Fourteenth Edition). For example, the disintegration properties at low pH are compared with those at a neutral region in accordance with the above Disintegration Test, and the disintegration properties are judged to be the same when the difference in content release starting time is within 30 minutes, preferably within 15 minutes, more preferably within 5 minutes.

As a water-soluble polysaccharide which can be the main component of the capsule, various materials such as pullulan, agar, carrageenan, starch and guar gum can be mentioned. Especially preferred is pullulan which is excellent in moldability when formed into a capsule, mechanical strength and solubility. Among them, preferred is, for example, a capsule having solubility at 37°C and mechanical strength equal to those of a gelatin capsule and in addition, being remarkably stable in a low moisture state and having excellent oxygen barrier properties as compared with a gelatin capsule. Such a capsule may, for example, be a capsule containing about 50 to about 99% by weight (preferably about 70 to about 90% by weight) of pullulan, such as NPcaps (manufactured by CAPSUGEL).

Further, the PEG-containing gelatin capsule is suitably a capsule in which polyethylene glycol is contained for the purpose of softening the capsule shell, and whose decrease in mechanical strength in a low moisture state, which is a drawback of a conventional gelatin capsule, is reduced. The amount of PEG is about 1 to about 30% by weight, preferably about 1 to about 10% by weight. Preferred is a gelatin capsule containing PEG having molecular weight of 200 to 20,000, especially preferred is a gelatin capsule containing PEG 4000 (average molecular weight of 2,600 to 3,800). Examples thereof include a gelatin capsule as disclosed in JP 6-11696 B. For example, a commercially available PEG-containing gelatin capsule (manufactured by Shionogi Qualicaps Co., Ltd.) may be used.

Further, it is possible to use a capsule comprising two types of capsule shells being stable in a low moisture state and having pH-independent disintegration properties in combination. For example, it is possible to use a capsule comprising a capsule shell comprising a water-soluble polysaccharide (such as pullulan) as the main component and a PEG-containing gelatin capsule shell in combination.

Herein, the "enteric coating" means a coating which is dissolved at ordinary pH of about 5.5, the "timed release coating" means a controlled film which quickly release an active ingredient by disintegration or dissolution of the coating layer after a lapse of desired certain time, and the "controlled release coating" dose not include an ordinary enteric coating but means a pH-dependent coating which is dissolved in a pH region which is different from that for an ordinary enteric coating, or a diffusion-controlled coating which is not soluble itself but releases an active ingredient through pores formed on the coating.

The "medicine unstable to moisture" to be used in the present invention usually includes medicines such as pharmaceutical active ingredients which undergo certain degeneration such as deterioration, coloring or degradation by moisture. The present invention is particularly preferably applicable to a medicine which is desirably stored or maintained in a low moisture state, among the above medicines. The above medicine includes that which undergoes a certain change such as coloring or deterioration usually at greater or equal to relative humidity of about 30%, especially greater or equal to that of about 40%.

Herein, the relative humidity (RH) is used as an index of a moisture content in an environment. Further, as an index of humidity in equilibrium of a substance or a composition at certain relative humidity (under humidity environment), the equilibrium relative humidity (ERH) is used. The equilibrium relative humidity is used as an index of a moisture content possessed by a substance or a composition, and represents a value corresponding to 100 times as much as the so-called water activity (Aw, which means mobile water, see Pharmaceutical Research, Vol.8, No. 3, 1991 (p 292-p 297), D.R. Heidemann and P.J. Jarosz). Accordingly, in the present invention, the moisture content of contents (the content in the capsule comprising the medicine and other carriers, etc.), the capsule shell and the entire capsule preparation comprising them may be replaced with the index "ERH" evaluated from the viewpoint of the water activity. This index ERH is applicable to not only capsule preparations but also tablets, granules, powders, liquid preparations, semisolid preparations, injectable preparations, etc. The equilibrium relative humidity can be measured, for example, by a Rotronic water activity measurement instrument (manufactured by Rotronic AG), but the measurement is not limited thereto.

In the present invention, the above-described capsule preparation containing a medicine unstable to moisture, which is stable event in a low moisture state is used for improvement in stability. In this case, the low moisture state means such a low moisture state that a gelatin capsule held therein will crack, and especially such a low moisture state that the loss on drying (LOD) of the capsule per se is at less or equal to about 10%, especially at less or equal to about 5%, in a humidity environment at relative humidity of at less or equal to about 35%, especially at relative humidity of at less or equal to about 25%. A stable capsule comprising as the main component a water-soluble polysaccharide such as pullulan or a capsule comprising as the main component polyethylene glycol-containing gelatin, which will not undergo degeneration such as cracking even in such a low moisture state, is preferably used.

The medicine per se contained in the capsule of the present invention and other capsule inclusions such as a carrier are preferably stored or maintained in a low moisture state at relative humidity of less or equal to about 35%, especially at relative humidity of less or equal to about 25%. Each of the capsule shell, the contents and the capsule preparation comprising them, is preferably in a humidity environment controlled to be at relative humidity of less or equal to about 35%, especially at a relative humidity of less or equal to about 25%, whereby a physicochemically stable capsule preparation can be produced.

Whether a capsule per se or a capsule preparation is stable in a low moisture state can be evaluated, for example, by a fracture ratio of a horizontally placed capsule which is pressurized, for example, at a rate of compression of 300 mm/min by means of an autograph (5,000 kgf load cell) in an equilibrium state, after stored at a given temperature (e.g. 25°C) at given relative humidity (e.g. 11%RH) for a certain period of time (e.g. about one week, preferably about two weeks).

Examples of the medicine unstable to moisture to be used in the present invention include procaine, atropine, aspirin, thiamin, penicillin, cephalosporin and one unstable to moisture among proton pump inhibitors (PPI), etc.

Examples of the imidazole type compound having a PPI activity to be used in the present invention include an imidazole type compound unstable to an acid represented by the following formula (I') such as lansoprazole or an optically active isomer thereof, especially a benzimidazole type compound unstable to an acid represented by the formula (I), especially the formula (Ia), a prodrug of an imidazole type compound relatively stable to an acid represented by the formula (II) mentioned hereinafter, a prodrug of known PPI, an optically active isomer thereof or a salt thereof. The capsule preparation or the like used these PPI as an active ingredient has excellent stability of the medicine per se and the preparation. 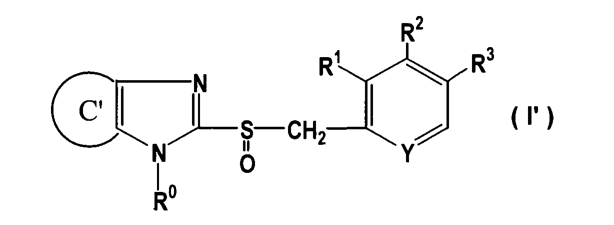 wherein the ring C' is an optionally substituted benzene or an optionally substituted aromatic mono-heterocyclic ring, R° is a hydrogen atom, an optionally substituted aralkyl group, an acyl group or an acyloxy group, each of R¹, R² and R³ which may be the same or different, and is a hydrogen atom, an optionally substituted alkyl group, an optionally substituted alkoxy group, or an optionally substituted amino group, and Y is a nitrogen atom or CH.

Among compounds represented by the above formula (I'), particularly a compound wherein the ring C' is an optionally substituted benzene ring is represented by the following formula (I).

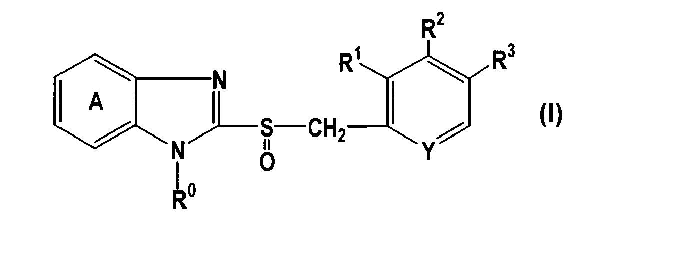

Namely, in the formula (I), the ring A is an optionally substituted benzene ring, and R⁰, R¹, R², R³ and Y are as defined in the above formula (I').

Preferred as the compound represented by the formula (I) is a compound wherein the ring A is a benzene ring which may have substituent(s) selected from a halogen atom, an optionally halogenated C₁₋₄ alkyl group, an optionally halogenated C₁₋₄ alkoxy group and a 5- or 6-membered heterocyclic group, R° is a hydrogen atom, an optionally substituted aralkyl group, an acyl group or an acyloxy group, R¹ is a C₁₋₆ alkyl group, a C₁₋₆ alkoxy group, a C₁₋₆ alkoxy-C₁₋₆ alkoxy group or a di-C₁₋₆ alkylamino group, R² is a hydrogen atom, a C₁₋₆ alkoxy-C₁₋₆ alkoxy group or an optionally halogenated C₁₋₆ alkoxy group, R³ is a hydrogen atom or a C₁₋₆ alkyl group, and Y is a nitrogen atom.

Particularly preferred is a compound represented by the formula (Ia): 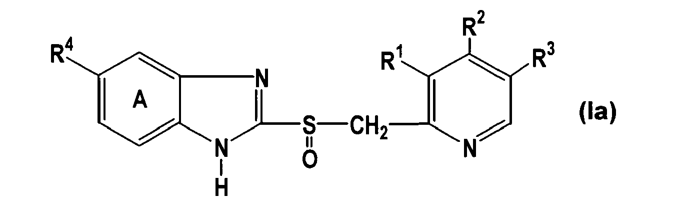 wherein R¹ is a C₁₋₃ alkyl group or a C₁₋₃ alkoxy group, R² is a C₁₋₃ alkoxy group which may be halogenated or substituted with a C₁₋₃ alkoxy group, R³ is a hydrogen atom or a C₁₋₃ alkyl group, and R⁴ is a hydrogen atom, an optionally halogenated C₁₋₃ alkoxy group, or a pyrrolyl group (e.g. 1-, 2- or 3-pyrrolyl group).

In the formula (Ia), particularly preferred is a compound wherein R¹ is a C₁₋₃ alkyl group, R² is an optionally halogenated C₁₋₃ alkoxy group, R³ is a hydrogen atom, and R⁴ is a hydrogen atom or an optionally halogenated C₁₋₃ alkoxy group.

Examples of the "substituent" in the "optionally substituted benzene ring" represented by the ring A in the compound represented by the above formula (I) (hereinafter referred to as compound (I), the same applies hereinafter) include a halogen atom, a cyano group, a nitro group, an optionally substituted alkyl group, a hydroxy group, an optionally substituted alkoxy group, an aryl group, an aryloxy group, a carboxy group, an acyl group, an acyloxy group, a 5- to 10-membered heterocyclic group, etc. and the benzene ring may be substituted with one to three or more substituents. When the number of substituents is 2 or more, the respective substituents may be the same or different. Among these substituents, a halogen atom, an optionally substituted alkyl group, an optionally substituted alkoxy group, etc. is preferred.

Examples of the halogen atom include fluorine, chlorine and bromine atoms, etc. Among them, fluorine is preferred.

Examples of the "alkyl group" in the "optionally substituted alkyl group" include C₁₋₇ alkyl groups (e.g. methyl, ethyl, propyl, isopropyl, butyl, isobutyl, sec-butyl, tert-butyl, pentyl, hexyl, heptyl groups, etc.). Examples of the "substituent" in the "optionally substituted alkyl group" include a halogen atom, a hydroxy group, C₁₋₆ alkoxy groups (e.g. methoxy, ethoxy, propoxy, butoxy, etc.), C₁₋₆ alkoxy-carbonyl groups (e.g. methoxycarbonyl, ethoxycarbonyl, propoxycarbonyl groups, etc.), a carbamoyl group, etc. and the number of these substituents may be 1 to 3 or more. When the number of substituents is 2 or more, the respective substituents may be the same or different.

Examples of the "alkoxy group" in the "optionally substituted alkoxy group" include C₁₋₆ alkoxy groups (e.g. methoxy, ethoxy, propoxy, isopropoxy, butoxy, isobutoxy, pentoxy, etc.) and the like. Examples of the "substituent" in the "optionally substituted alkoxy group" include the same "substituents" as those for the above "substituent" in the "optionally substituted alkyl group", and the number of substituents is also the same as defined above.

Examples of the "aryl group" include C₆₋₁₄ aryl groups (e.g. phenyl, 1-naphthyl, 2-naphthyl, biphenyl, 2-anthryl groups, etc.) and the like.

Examples of the "aryloxy group" include C₆₋₁₄ aryloxy groups (e.g. phenyloxy, 1-naphthyloxy, 2-naphthyloxy groups, etc.) and the like.

Examples of the "acyl group" include formyl, alkylcarbonyl, alkoxycarbonyl, carbamoyl, alkylcarbamoyl, alkylsulfinyl, alkylsulfonyl groups, and the like.

Examples of the "alkylcarbonyl group" include C₁₋₆ alkyl-carbonyl groups (e.g. acetyl, propionyl groups, etc.) and the like.

Examples of the "alkoxycarbonyl group" include C₁₋₆ alkoxy-carbonyl groups (e.g. methoxycarbonyl, ethoxycarbonyl, propoxycarbonyl, butoxycarbonyl groups, etc.) and the like.

Examples of the "alkylcarbamoyl group" include N-C₁₋₆ alkyl-carbamoyl groups (e.g. methylcarbamoyl, ethylcarbamoyl groups, etc.), N,N-di-C₁₋₆ alkyl-carbamoyl groups (e.g. N,N-dimethycarbamoyl, N,N-diethylcarbamoyl groups, etc.) and the like.

Examples of the "alkylsulfinyl group" include C₁₋₇ alkylsulfinyl groups (e.g. methylsulfinyl, ethylsulfinyl, propylsulfinyl, isopropylsulfinyl groups, etc.).

Examples of the "alkylsulfonyl group" include C₁₋₇ alkylsulfonyl groups (e.g. methylsulfonyl, ethylsulfonyl, propylsulfonyl, isopropylsulfonyl groups, etc.).

Examples of the "acyloxy group" include an alkylcarbonyloxy group, an alkoxycarbonyloxy group, a carbamoyloxy group, an alkylcarbamoyloxy group, an alkylsulfinyloxy group, an alkylsulfonyloxy group and the like.

Examples of the "alkylcarbonyloxy group" include C₁₋₆ alkyl-carbonyloxy groups (e.g. acetyloxy, propionyloxy groups, etc.) and the like.

Examples of the "alkoxycarbonyloxy group" include C₁₋₆ alkoxy-carbonyloxy groups (e.g. methoxycarbonyloxy, ethoxycarbonyloxy, propoxycarbonyloxy, butoxycarbonyloxy groups, etc.) and the like.

Examples of the "alkylcarbamoyloxy group" include C₁₋₆ alkyl-carbamoyloxy groups (e.g. methylcarbamoyloxy, ethylcarbamoyloxy groups, etc.) and the like.

Examples of the "alkylsulfinyloxy group" include C₁₋₇ alkylsulfinyloxy groups (e.g. methylsulfinyloxy, ethylsulfinyloxy, propylsulfinyloxy, isopropylsulfinyloxy groups, etc.) and the like.

Examples of the "alkylsulfonyloxy group" include C₁₋₇ alkylsulfonyloxy groups (e.g. methylsulfonyloxy, ethylsulfonyloxy, propylsulfonyloxy, isopropylsulfonyloxy groups, etc.).

Examples of the "5- to 10-membered heterocyclic group" include 5- to 10-membered (preferably 5- to 6-membered) heterocyclic groups containing 1 or more (for example, 1 to 3) hetero atom(s) selected from a nitrogen atom, a sulfur atom and an oxygen atom in addition to carbon atoms, and specific examples thereof include a 2- or 3-thienyl group, a 2-, 3- or 4-pyridyl group, a 2- or 3-furyl group, a 1-, 2- or 3-pyrrolyl group, a 2-, 3-, 4-, 5- or 8-quinolyl group, a 1-, 3-, 4- or 5-isoquinolyl group, and a 1-, 2- or 3-indolyl group and the like. Among them, preferred is a 5- or 6-membered heterocyclic group such as a 1-, 2- or 3-pyrrolyl group, etc.

Preferably, the ring A is a benzene ring optionally having 1 or 2 substituent(s) selected from a halogen atom, an optionally halogenated C₁₋₄ alkyl group, an optionally halogenated C₁₋₄ alkoxy group and a 5- or 6-membered heterocyclic group.

In the above formula (I'), examples of the "aromatic mono-heterocyclic ring" in the "optionally substituted aromatic mono-heterocyclic ring" represented by the ring C' include 5- to 6-membered aromatic monocyclic heterocyclic rings such as furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazan, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine, etc. The "aromatic mono-heterocyclic ring" represented by the ring C' is especially preferably the "optionally substituted benzene ring" or the "optionally substituted pyridine ring" represented by the above ring A. The "aromatic mono-heterocyclic ring" represented by the ring C' may have 1 to 4 substituent(s) selected from the same substituents as those for the "optionally substituted benzene ring" represented by the above ring A at possible position(s).

The position at which the "aromatic mono-heterocyclic ring" in the "optionally substituted aromatic mono-heterocyclic ring" is attached to the imidazole moiety is not particularly limited.

In the above formulae (I') and (I), examples of the "aralkyl group" in the "optionally substituted aralkyl group" represented by R⁰ include C₇₋₁₆ aralkyl groups (e.g. C₆₋₁₀ aryl-C₁₋₆ alkyl groups such as benzyl, phenethyl, etc.). Examples of the "substituent" in the "optionally substituted aralkyl group" include the same substituents as the "substituents" for the above "optionally substituted alkyl group", and the number of substituents is 1 to 4 or more. When the number of substituents is 2 or more, the respective substituents may be the same or different.

Examples of the "acyl group" represented by R° include the "acyl group" disclosed as the substituents for the above ring A.

Examples of the "acyloxy group" represented by R° include the "acyloxy group" disclosed as the substituents for the above ring A.

Preferred R° is a hydrogen atom.

In the above formula (I') and (I), examples of the "optionally substituted alkyl group" represented by each of R¹, R² and R³ include the above "optionally substituted alkyl group groups" described as the substituents for the above ring A.

Examples of the "optionally substituted alkoxy group" represented by each of R¹, R² and R³ include the above "optionally substituted alkoxy groups" described as the substituents for the above ring A.

Examples of the "optionally substituted amino group" represented by each of R¹, R² and R³ include an amino group, mono-C₁₋₆ alkylamino groups (e.g. methylamino, ethylamino, etc.), mono-C₆₋₁₄ arylamino groups (e.g. phenylamino, 1-naphthylamino, 2-naphthylamino, etc.), di-C₁₋₆ alkylamino groups (e.g. dimethylamino, diethylamino, etc.), di-C₆₋₁₄ arylamino groups (e.g. diphenylamino, etc.) and the like.

Preferred R¹ is a C₁₋₆ alkyl group, a C₁₋₆ alkoxy group, a C₁₋₆ alkoxy-C₁₋₆ alkoxy group, or a di-C₁₋₆ alkylamino group. More preferred R² is a C₁₋₃ alkyl group or a C₁₋₃ alkoxy group.

Preferred R² is a hydrogen atom, a C₁₋₆ alkoxy-C₁₋₆ alkoxy group or an optionally halogenated C₁₋₆ alkoxy group. More preferred is a C₁₋₃ alkoxy group which may be halogenated or substituted with a C₁₋₃ alkoxy group.

Preferred R³ is a hydrogen atom, or a C₁₋₆ alkyl group. More preferred R³ is a hydrogen atom or a C₁₋₃ alkyl group (particularly a hydrogen atom).

Preferred Y is a nitrogen atom.

Specific examples of the compound (I) include the following compounds:
2-[[[3-methyl-4-(2,2,2-trifluoroethoxy)-2-pyridinyl]methyl]sulfinyl]-1H-imidazole (lansoprazole), 2-[[(3,5-dimethyl-4-methoxy-2-pyridinyl)methyl]sulfinyl]-5-methoxy-1H-benzimidazole, 2-[[[4-(3-methoxypropoxy)-3-methyl-2-pyridinyl]methyl]sulfinyl]-1H-benzimidazole sodium salt, 5-difluoromethoxy-2-[[(3,4-dimethoxy-2-pyridinyl)methyl]sulfinyl]-1H-benzimidazole, etc.

Among these compounds, in particular, lansoprazole, that is, 2-[[[3-methyl-4-(2,2,2-trifluoroethoxy)-2-pyridinyl]methyl]sulfinyl]-1H-benzimidazole is preferred.

In addition to the above-described benzimidazole type PPI compounds, the present invention is also preferably applicable to imidazopyridine type PPI compounds. Examples of the imidazopyridine type PPI compounds include tenatoprazole, etc.

The above compounds (I'), (I) and (Ia) may be racemates or optically active isomers such as R-isomer and S-isomer. For example, optically active isomers of lansoprazole, i.e. optically active isomers such as (R)-2-[[[3-methyl-4-(2,2,2-trifluoroethoxy)-2-pyridinyl]methyl]sulfinyl]-1H-benzimidazole (hereinafter sometimes referred to as lansoprazole R isomer) and (S)-2-[[[3-methyl-4-(2,2,2-trifluoroethoxy)-2-pyridinyl]methyl]sulfinyl]-1H-benzimidazole (hereinafter sometimes referred to as lansoprazole S isomer) are particularly suitable in the present invention. Usually, lansoprazole, lansoprazole R isomer, lansoprazole S isomer, etc. are preferably in the form of crystals. However, not only crystalline compounds but also amorphous compounds can be used.

As a salt of the compound (I') and the compounds (I) and (Ia), a pharmaceutically acceptable salt is preferred, and examples thereof include a salt with an inorganic base, a salt with an organic base, a salt with a basic amino acid and the like.

Preferred examples of the salt with an inorganic base include alkali metal salts such as a sodium salt, a potassium salt, etc.; alkaline earth metal salts such as a calcium salt, a magnesium salt, etc.; an ammonium salt and the like.

Preferred examples of the salt with an organic base include salts with alkylamines (trimethylamine, triethylamine, etc.), heterocyclic amines (pyridine, picoline, etc.), alkanolamines (ethanolamine, diethanolamine, triethanolamine, etc.), dicyclohexylamine, N,N'-dibenzylethylenediamine and the like. Preferred specific examples of the salt with an organic base include salts with trimethylamine, triethylamine, pyridine, picoline, 2,6-lutidine, ethanolamine, diethanolamine, triethanolamine, tromethamine [tris(hydroxymethyl)methylamine], t-butylamine, cyclohexylamine, dicyclohexylamine, N,N'-dibenzylethylenediamine and the like.

Preferred examples of the salt with a basic amino acid include salts with arginine, lysine, ornithine and the like.

Among these salts, an alkali metal salt or an alkaline earth metal salt is preferred. Especially, a sodium salt is preferred.

The compounds (I'), (I) and (Ia) can be prepared by known per se methods, for example, the methods described in JP 61-50978 A, U.S. Patent No.4,628,098, JP 10-195068 A, WO 98/21201, JP 52-62275 A, JP 54-141783 A and the like, or similar methods. The optically active compound (I) can be obtained by an optical resolving method (fractionating recystallization method, chiral column method, diastereomer method, method using microorganisms or enzymes and the like), asymmetric oxidation method and the like. Lansoprazole R isomer can also be prepared by a process described, for example, in WO 00/78745, WO 01/83473 and the like.

As the imidazole compound having gastric acid secretion inhibiting activity used in the present invention, lansoprazole, omeprazole, rabeprazole, pantoprazole, leminoprazole, tenatoprazole (TU-199) and the like and optically active isomers thereof as well as pharmaceutically acceptable salts are preferred, and lansoprazole or an optically active isomer thereof, in particular, an R isomer is more preferred. Lansoprazole or an optically active isomer thereof, in particular, an R isomer is preferably a crystalline compound but may be an amorphous compound. The present invention is also preferably applicable to prodrugs of these PPI unstable to moisture.

Preferred examples of the prodrugs include, not only prodrugs included in the compounds (I) and (I') but also prodrugs as disclosed in WO 03/105845, i.e. compounds represented by the following formula (II) and prodrugs as disclosed in WO 02/30920, WO 03/270982 and the like.

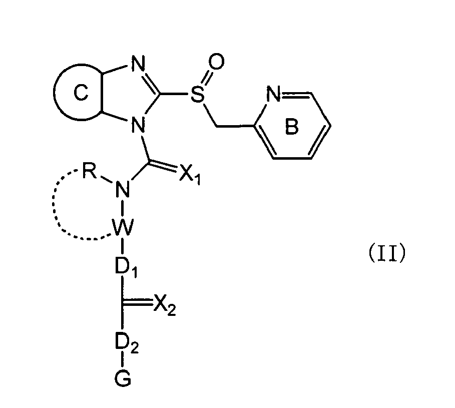

In the compound represented by the above formula (II) (hereinafter referred to as compound (II)), the ring B is an "optionally substituted pyridine ring".

The pyridine ring of the "optionally substituted pyridine ring" represented by the ring B may have 1 to 4 substituent(s) at possible position(s). Example of the substituent include halogen atoms (e.g. fluorine, chlorine, bromine, iodine, etc.), optionally substituted hydrocarbon groups (e.g. C₁₋₆ alkyl groups such as a methyl group, an ethyl group, a n-propyl group, etc.), optionally substituted amino groups (e.g. amino, amino groups mono-substituted or di-substituted with C₁₋₆ alkyl groups such as methylamino, dimethylamino, ethylamino, diethylamino groups, etc.), amide groups (e.g. C₁₋₃ acylamino groups such as formamide, acetamide, etc.), optionally substituted lower alkoxy groups(e.g. C₁₋₆ alkoxy groups such as methoxy, ethoxy, 2,2,2-trifluoroethoxy, 3-methyoxypropoxy, etc.), lower alkylenedioxy groups (e.g. C₁₋₃ alkylenedioxy groups such as methylenedioxy, ethylenedioxy, etc.).

Example of the substituent of the optional substituent in the "optionally substituted pyridine ring" represented by the ring B include halogen atoms (e.g. fluorine, chlorine, bromine, iodine groups, etc.), lower alkyl groups (e.g. C₁₋₆ alkyl groups such as methyl, ethyl, propyl groups, etc.), lower alkenyl groups (e.g. C₂₋₆ alkenyl groups such as vinyl, allyl groups, etc.), lower alkynyl groups (e.g. C₂₋₆ alkynyl groups such as ethinyl, propargyl groups, etc.), cycloalkyl groups (e.g. C₃₋₈ cycloalkyl groups such as cyclopropyl, cyclobutyl, cyclopentyl, cyclohexyl groups, etc.), lower alkoxy groups (e.g. C₁₋₆ alkoxy groups such as methoxy, ethoxy groups, etc.), a nitro group, a cyano group, a hydroxy group, a thiol group, a carboxyl group, lower alkanoyl groups (e.g. formyl; C₁₋₆ alkyl-carbonyl groups such as acetyl, propionyl, butyryl groups, etc.), lower alkanoyloxy groups (e.g. formyloxy; C₁₋₆ alkyl-carbonyloxy groups such as acetyloxy, propionyloxy groups, etc.), lower alkoxycarbonyl groups (e.g. C₁₋₆ alkoxy-carbonyl groups such as methoxycarbonyl, ethoxycarbonyl, propoxycarbonyl groups, etc.), aralkyloxycarbonyl groups (e.g. C₇₋₁₁ aralkyloxy-carbonyl groups such as a benzyloxycarbonyl group, etc.), aryl groups (e.g. C₆₋₁₄ aryl groups such as phenyl, naphthyl groups, etc.), aryloxy groups (e.g. C₆₋₁₄ aryloxy groups such as phenyloxy, naphthyloxy groups, etc.), arylcarbonyl groups (e.g. C₆₋₁₄ aryl-carbonyl groups such as benzoyl, naphthoyl groups, etc.), arylcarbonyloxy groups (e.g.C₆₋₁₄ aryl-carbonyloxy groups such as benzoyloxy, naphthoyloxy groups, etc.), optionally substituted carbamoyl groups (e.g. carbamoyl; carbamoyl groups mono-substituted or di-substituted with C₁₋₆ alkyl groups such as methylcarbamoyl, dimethylcarbamoyl groups, etc.), optionally substituted amino groups (e.g. amino; amino groups mono-substituted or di-substituted with C₁₋₆ alkyl groups such as methylamino, dimethylamino, ethylamino, diethylamino groups, etc.), and the like. The number of substituents and the substitution positions are not particularly limited.

The number and the substitution positions of substituents in the "optionally substituted pyridine ring" represented by the ring B are not particularly limited, but preferably the pyridine ring is substituted with 1 to 3 the above substituent(s) at any of 3-, 4- and 5-positions.

The "optionally substituted pyridine ring" represented by the ring B is preferably 3-methyl-4-(2,2,2-trifluoroethoxy)-2-pyridyl.

In the present invention, the ring C is an "optionally substituted benzene ring" or an "optionally substituted aromatic mono-heterocyclic ring" condensed to the imidazole moiety, and the former is preferred.

The benzene ring in the "optionally substituted benzene ring" represented by the ring C may have 1 to 4 substituent(s) at possible position(s). Examples of the substituent include halogen atoms (e.g. fluorine, chlorine, bromine, iodine, etc.), optionally substituted hydrocarbon groups (e.g. C₁₋₆ alkyl groups such as a methyl group, an ethyl group, a n-propyl group, etc.),optionally substituted amino groups (e.g. amino, amino groups mono-substituted or di-substituted with C₁₋₆ alkyl groups such as methylamino, dimethylamino, ethylamino, diethyl amino groups, etc.), amide groups (e.g. C₁₋₃ acylamino groups such as formamide, acetamide, etc.), optionally substituted lower alkoxy groups (e.g. C₁₋₆ alkoxy groups such as methoxy, ethoxy, difluoromethoxy groups, etc.), lower alkylenedioxy groups (e.g. C₁₋₃ alkylenedioxy groups such as methylenedioxy, ethylenedioxy, etc.), and the like.

Examples of the substituent of the optional substituent in the "optionally substituted benzene ring" represented by the ring C include halogen atoms (e.g. fluorine, chlorine, bromine, iodine, etc.), lower alkyl groups (e.g. C₁₋₆ alkyl groups such as methyl, ethyl, propyl groups, etc.), lower alkenyl groups (e.g. C₂₋₆ alkenyl groups such as vinyl, allyl groups, etc.), lower alkynyl groups (e.g. C₂₋₆ alkynyl groups such as ethinyl, propargyl groups, etc.), cycloalkyl groups (e.g. C₃₋₈ cycloalkyl groups such as cyclopropyl, cyclobutyl, cyclopentyl, cyclohexyl groups, etc.), lower alkoxy groups (e.g. C₁₋₆ alkoxy groups such as methoxy, ethoxy groups, etc.), a nitro group, a cyano group, a hydroxy group, a thiol group, a carboxyl group, lower alkanoyl groups (e.g. formyl; C₁₋₆ alkyl-carbonyl groups such as acetyl, propionyl, butyryl groups, etc.), lower alkanoyloxy groups (e.g. formyloxy; C₁₋₆ alkyl-carbonyloxy groups such as acetyloxy, propionyloxy groups, etc.), lower alkoxycarbonyl groups (e.g. C₁₋₆ alkoxy-carbonyl groups such as methoxycarbonyl, ethoxycarbonyl, propoxycarbonyl groups, etc.), aralkyloxycarbonyl groups (e.g. C₇₋₁₇ aralkyloxy-carbonyl groups such as a benzyloxycarbonyl group, etc.), aryl groups (e.g. C₆₋₁₄ aryl groups such as phenyl, naphthyl groups, etc.), aryloxy groups (e.g. C₆₋₁₄ aryloxy groups such as phenyloxy, naphthyloxy groups, etc.), arylcarbonyl groups (e.g. C₆₋₁₄ aryl-carbonyl groups such as benzoyl, naphthoyl groups, etc.), arylcarbonyloxy groups (e.g. C₆₋₁₄ acryl-carbonyloxy groups such as benzoyloxy, naphthoyloxy, etc.), optionally substituted carbamoyl groups (e.g. carbamoyl; carbamoyl groups mono-substituted or di-substituted with C₁₋₆ alkyl groups such as methylcarbamoyl, dimethylcarbamoyl, etc.), optionally substituted amino groups (e.g. amino; amino groups mono-substituted or di-substituted with C₁₋₆ alkyl groups such as methylamino, dimethylamino, ethylamino, diethylamino groups, etc.). The number of substituents and the substitution positions are not particularly limited.

The "optionally substituted benzene ring" represented by the ring C is preferably a benzene ring.

Examples of the "aromatic mono-heterocyclic ring" in the "optionally substituted aromatic mono-heterocyclic ring" represented by the ring C include 5- to 6-membered aromatic monocyclic heterocyclic rings such as furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazan, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine, etc. The "aromatic mono-heterocyclic ring" represented by the ring C is especially preferably a pyridine ring. It may have 1 to 4 substituent(s) which are the same as those for the "optionally substituted benzene ring" represented by the ring C at possible position(s).

The position at which the "aromatic mono-heterocyclic ring" in the "optionally substituted aromatic mono-heterocyclic ring" is condensed to the imidazole moiety is not particularly limited.

In the present invention, X₁ and X₂ are an oxygen atom and a sulfur atom, respectively. Preferably both X₁ and X₂ are oxygen atoms.

In the present invention, W is an "optionally substituted bivalent chain hydrocarbon group" or a bivalent group represented by the formula

**―W**_{**1**}**―Z―W**_{**2**}**―**

wherein each of W₁ and W₂ is a "bivalent chain hydrocarbon group" or a bond, Z is an "optionally substituted bivalent hydrocarbon group", an "optionally substituted bivalent heterocyclic group", an oxygen atom, SOₙ (wherein n is 0, 1 or 2), or >N-E (wherein E is a hydrogen atom, an optionally substituted hydrocarbon group, an optionally substituted heterocyclic group, a lower alkanoyl group, a lower alkoxycarbonyl group, an aralkyloxycarbonyl group, a thiocarbamoyl group, a lower alkylsulfinyl group, a lower alkylsulfonyl group, a sulfamoyl group, a mono-lower alkylsulfamoyl group, a di-lower alkylsulfamoyl group, an arylsulfamoyl group, an arylsulfinyl group, an arylsulfonyl group, an arylcarbonyl group, or an optionally substituted carbamoyl group), provided that, when Z is an oxygen atom, SOₙ or >N-E, each of W₁ and W₂ is a "bivalent chain hydrocarbon group". Particularly, W is preferably a "optionally substituted bivalent chain hydrocarbon group".

Examples of the "bivalent chain hydrocarbon group" in the "optionally substituted bivalent chain hydrocarbon group" represented by W and the "bivalent chain hydrocarbon group" represented by W₁ and W₂ include C₁₋₆ alkylene groups (e.g. methylene, ethylene, trimethylene, etc.), C₂₋₆ alkenylene groups (e.g. ethenylene, etc.), C₂₋₆ alkynylene groups (e.g. ethynylene, etc.), and the like. The bivalent chain hydrocarbon group represented by W may have 1 to 6 the substituent(s) selected from the same substituents as those in the "optionally substituted benzene ring" at possible position(s).

The "bivalent chain hydrocarbon group" in the "optionally substituted bivalent chain hydrocarbon group" represented by W and the "bivalent chain hydrocarbon group" represented by W₁ and W₂ is preferably a methylene group or an ethylene group. W is particularly preferably an ethylene group. When Z is an oxygen atom, SOₙ or >N-E (wherein n and E are as defined above), the "bivalent chain hydrocarbon group" represented by W₁ is preferably a hydrocarbon group having 2 or more carbon atoms.

Examples of the "hydrocarbon ring" in the "optionally substituted bivalent hydrocarbon group" represented by Z include alicyclic hydrocarbon rings, aromatic hydrocarbon rings, and the like, and preferred examples include C₃₋₁₆ hydrocarbon rings, and it may have 1 to 4 substituent(s) selected from the same substituents as those in the "optionally substituted benzene ring" represented by the ring C at possible positions. Examples of the hydrocarbon ring include cycloalkane, cycloalkene, arene, and the like.

Examples of the "cycloalkane" in the "optionally substituted bivalent hydrocarbon group" represented by Z include lower cycloalkanes, etc., and C₃₋₁₀ cycloalkanes such as cyclopropane, cyclobutane, cyclopentane, cyclohexane, cycloheptane, cyclooctane, bicyclo[2.2.1]heptane, adamantane, etc., are generally used.

Examples of the "cycloalkene" in the "optionally substituted bivalent hydrocarbon group" represented by Z include lower cycloalkenes, etc., and C₄₋₉ cycloalkenes such as cyclopropene, cyclobutene, cyclopentene, cyclohexene, cycloheptene, cyclooctene, etc., are generally used.

Examples of the "arene" in the "optionally substituted bivalent hydrocarbon group" represented by Z include C₆₋₁₄ arenes such as benzene, naphthalene, phenanthrene, etc., and, for example, phenylene is generally used.

Examples of the "heterocyclic ring" in the "optionally substituted bivalent heterocyclic group" represented by Z include 5- to 12-membered "aromatic heterocyclic ring" and "saturated or unsaturated non-aromatic heterocyclic ring" containing 1 or more (preferably 1 to 4, more preferably 1 or 2) of one to three types (preferably one or two) hetero atom(s) selected from an oxygen atom, a sulfur atom and a nitrogen atom as an atom (ring atom) constituting the ring, and it may have 1 to 4 the substituent(s) selected from the same substituents as those in the "optionally substituted benzene ring" represented by the ring C at possible position(s).

Examples of the "aromatic heterocyclic ring" in the "optionally substituted bivalent heterocyclic group" represented by Z include aromatic monocyclic heterocyclic rings, aromatic condensed heterocyclic rings, etc.

Examples of the "aromatic monocyclic heterocyclic ring" include 5- to 6-membered aromatic monocyclic heterocyclic rings such as furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazan, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine, etc.

Examples of the "aromatic condensed heterocyclic ring" include 8- to 12-membered aromatic condensed heterocyclic rings such as benzofuran, isobenzofuran, benzothiophene, isobenzothiophene, indole, isoindole, 1H-indazole, benzimidazole, benzoxazole, 1,2-benzisoxazole, benzothiazole, 1,2-benzisothiazole, 1H-benzotriazole, quinoline, isoquinoline, cinnoline, quinazoline, quinoxaline, phthalazine, naphthyridine, purine, pteridine, carbazole, carboline, acridine, phenoxazine, phenothiazine, phenazine, phenoxathiine, thianthrene, phenanthridine, phenanthroline, indolizine, pyrrolo[1,2-b] pyridazine, pyrazolo[1,5-a]pyridine, imidazo[1,2-a]pyridine, imidazo[1,5-a]pyridine, imidazo[1,2-b]pyridazine, imidazo[1,2-a]pyrimidine, 1,2,4-triazolo[4,3-a]pyridazine, 1,2,4-triazolo[4,3-b]pyridazine, etc.

Examples of the "saturated or unsaturated non-aromatic heterocyclic ring" in the "optionally substituted bivalent heterocyclic group" represented by Z include 3- to 8-membered (preferably 5 to 6-membered) saturated or unsaturated (preferably saturated) non-aromatic heterocyclic ring (aliphatic heterocyclic ring) such as oxirane, azetidine, oxetane, thietane, pyrrolidine, tetrahydrofuran, tetrahydrothiophene, piperidine, tetrahydropyran, tetrahydrothiopyran, morpholine, thiomorpholine, piperazine, azepane, oxepane, thiene, oxazepane, thiazepane, azocane, oxocane, thiocane, oxazocane, thiazocane, etc. These may optionally be oxo-substituted, and examples include 2-oxoazetidine, 2-oxopyrrolidine, 2-oxopiperidine, 2-oxoazepane, 2-oxoazocane, 2-oxotetrahydrofuran, 2-oxotetrahydropyran, 2-oxotetrahydrothiophene, 2-oxothiane, 2-oxopiperazine, 2-oxooxepane, 2-oxooxazepane, 2-oxothiepane, 2-oxothiazepane, 2-oxooxocane, 2-oxothiocane, 2-oxooxazocane, 2-oxothiazocane, etc.

The two bonds from the "hydrocarbon ring group" in the "optionally substituted bivalent hydrocarbon ring group" or the "heterocyclic group" in the "optionally substituted hydrocarbon group" represented by Z may be located at any possible positions.

The "optionally substituted hydrocarbon group" and the "optionally substituted heterocyclic group" represented by E are as defined hereinafter.

Examples of the "lower alkanoyl group" represented by Z include formyl and C₁₋₆ alkyl-carbonyl groups such as acetyl, propionyl, butyryl, isobutyryl, etc.

Examples of the "lower alkoxycarbonyl group" represented by E include C₁₋₆ alkoxy-carbonyl groups such as methoxycarbonyl, ethoxycarbonyl, propoxycarbonyl, butoxycarbonyl, etc.

Examples of the "aralkyloxycarbonyl" represented by E include C₇₋₁₁ aralkyloxy-carbonyl groups such as benzyloxycarbonyl, etc.

Examples of the "lower alkylsulfinyl group" represented by E include C₁₋₆ alkylsulfinyl groups such as methylsulfinyl, ethylsulfinyl, etc.

Examples of the "lower alkylsulfonyl group" represented by E include C₁₋₆ alkylsulfonyl groups such as methylsulfonyl, ethylsulfonyl, etc.

Examples of the "mono-lower alkylsulfamoyl group" represented by E include mono-C₁₋₆ alkylsulfamoyl groups such as methylsulfamoyl, ethylsulfamoyl, etc.

Examples of the "di-lower alkylsulfamoyl group" represented by E include di-C₁₋₆ alkylsulfamoyl groups such as dimethylsulfamoyl, diethylsulfamoyl, etc.

Examples of the "arylsulfamoyl group" represented by E include C₆₋₁₀ arylsulfamoyl groups such as phenylsulfamoyl, naphthylsulfamoyl, etc.

Examples of the "arylsulfinyl group" represented by E include C₆₋₁₀ arylsulfinyl groups such as phenylsulfinyl, naphthylsulfinyl, etc.

Examples of the "arylsulfonyl group" represented by E include C₆₋₁₀ arylsulfonyl groups such as phenylsulfonyl, naphthylsulfonyl, etc.

Examples of the "arylcarbonyl group" represented by E include C₆₋₁₀ aryl-carbonyl groups such as benzoyl, naphthoyl, etc.

Examples of the "optionally substituted carbamoyl group" represented by E include a group represented by the formula -CONR₂R₃ (wherein each of R₂ and R₃ is a hydrogen atom, an optionally substituted hydrocarbon group or an optionally substituted heterocyclic group. Further, R₂ and R₃ may form a ring together with the adjacent nitrogen atom), etc.

In the present invention, R is an "optionally substituted hydrocarbon group" or an "optionally substituted heterocyclic group", and R can be bonded to W, and it is particularly preferably a C₁₋₆ optionally substituted hydrocarbon group, especially a lower (C₁₋₆) alkyl group. The "optionally substituted hydrocarbon group" and the "optionally substituted heterocyclic group" represented by R are as defined hereinafter. Further, the case where R is bonded to W will be described in detail hereinafter.

In the present invention, each of D₁ and D₂ is a bond, an oxygen atom, a sulfur atom or >NR₁, wherein R₁ is a hydrogen atom or an optionally substituted hydrocarbon group. However, in the present invention, the case where both D₁ and D₂ are bonds is excluded. Preferably each of D₁ and D₂ is a bond or an oxygen atom, particularly preferably D₁ is an oxygen atom and D₂ is an oxygen atom or a bond. The "optionally substituted hydrocarbon group" represented by R₁ is as defined hereinafter.

In the present invention, G is an "optionally substituted hydrocarbon group" or an "optionally substituted heterocyclic group", and it is preferably a C₁₋₆ optionally substituted hydrocarbon group or an optionally substituted saturated heterocyclic group containing 1 to 4 hetero atom(s) selected from an oxygen atom, a nitrogen atom and a sulfur atom as the ring constituent atom. Especially, G is preferably a C₁₋₆ optionally substituted hydrocarbon group or an optionally substituted saturated oxygen-containing heterocyclic group which may further contain 1 to 3 hetero atom(s) selected from an oxygen atom, a nitrogen atom and a sulfur atom as the ring constituent atom. The "optionally substituted hydrocarbon group" and the "optionally substituted heterocyclic group" represented by G are as defined hereinafter.

Examples of the "hydrocarbon group" in the "optionally substituted hydrocarbon group" represented by the above E, R, R₁ and G include saturated or unsaturated aliphatic hydrocarbon groups, saturated or unsaturated alicyclic hydrocarbon groups, saturated or unsaturated alicyclic-aliphatic hydrocarbon groups, aromatic hydrocarbon groups, aromatic-saturated or unsaturated alicyclic hydrocarbon groups, etc. Preferred is a C₁₋₁₆ hydrocarbon group, more preferred is a C₁₋₆ hydrocarbon group. Specific examples include alkyl groups, alkenyl groups, alkynyl groups, cycloalkyl groups, cycloalkenyl groups, cycloalkylalkyl groups, cycloalkenylalkyl groups, aryl groups, arylalkyl groups, etc.

Preferred examples of the "alkyl group" include lower alkyl groups (C₁₋₆ alkyl groups), and C₁₋₆ alkyl groups such as methyl, ethyl, propyl, isopropyl, butyl, isobutyl, sec-butyl, tert-butyl, pentyl, 1-ethylpropyl, hexyl, etc. are generally used. As R, a lower alkyl group (C₁₋₆ alkyl group) is preferred, and a methyl group is particularly preferred.

Preferred examples of the "alkenyl group" include lower alkenyl groups, and C₂₋₇ alkenyl groups such as vinyl, 1-propenyl, allyl, isopropenyl, butenyl, isobutenyl, 2,2-dimethyl-pent-4-enyl, etc. are generally used.

Preferred examples of the "alkynyl group" include lower alkynyl groups, and C₂₋₆ alkynyl groups such as ethinyl, propargyl, 1-propynyl, etc. are generally used.

Preferred examples of the "cycloalkyl group" include lower cycloalkyl groups, and C₃₋₁₀ cycloalkyl groups such as cyclopropyl, cyclobutyl, cyclopentyl, cyclohexyl, cycloheptyl, cyclooctyl, bicycle[2.2.1]heptanyl, adamantyl, etc. are generally used.

Preferred examples of the "cycloalkenyl group" include lower cycloalkenyl groups, and C₃₋₁₀ cycloalkenyl groups such as cyclopropenyl, cyclobutenyl, cyclopentenyl, cyclohexenyl, cycloheptenyl, cyclooctenyl, bicycle[2.2.1]hept-5-en-2-yl, etc. are generally used.

Preferred examples of the "cycloalkylalkyl group" include lower cycloalkylalkyl groups, and C₄₋₉ cycloalkylalkyl groups such cyclopropylmethyl, cyclopropylethyl, cyclobutylmethyl, cyclopentylmethyl, cyclohexylmethyl, cyclohexylethyl, etc. are generally used.

Preferred examples of the "cycloalkenylalkyl group" include lower cycloalkenylalkyl groups, and C₄₋₉ cycloalkenylalkyl groups such as cyclopentenylmethyl, cyclohexenylmethyl, cyclohexenylethyl, cyclohexenylpropyl, cycloheptenylmethyl, cycloheptenylethyl, bicycle[2.2.1]hept-5-en-2-ylmethyl, etc. are generally used.

Preferred examples of the "aryl group" include C₆₋₁₄ aryl groups such as phenyl, 1-naphtyl, 2-naphthyl, biphenyl, 2-anthryl, etc., and, for example, a phenyl group is generally used.

The "arylalkyl group" has the above defined "aryl group" as the aryl moiety and the above defined "alkyl group" as the alkyl moiety. Particularly preferred examples include C₆₋₁₄ aryl-C₁₋₆ alkyl groups, and, for example, benzyl, phenethyl, etc. are generally used.

Examples of the optional substituent of the "hydrocarbon group" in the "optionally substituted hydrocarbon group" represented by the above E, R, R₁ and G include halogen atoms (e.g. fluorine, chlorine, bromine, iodine, etc.), a nitro group, a cyano group, a hydroxyl group, a thiol group, a sulfo group, a sulfino group, a phosphono group, optionally halogenated lower alkyl groups (e.g. C₁₋₆ alkyl such as methyl, ethyl, propyl, isopropyl, butyl, isobutyl, sec-butyl, tert-butyl, pentyl, 1-ethylpropyl, hexyl, etc., mono-, di- or tri-halogeno-C₁₋₆ alkyl groups such as chloromethyl, dichloromethyl, trichloromethyl, fluoromethyl, difluoromethyl, trifluoromethyl, 2-bromoethyl, 2,2,2-trifluoroethyl, pentafluoroethyl, 3,3,3-trifluoropropyl, 4,4,4-trifluorobutyl, 5,5,5-trifluoropentyl, 6,6,6-trifluorohexyl, etc), an oxo group, an amidino group, an imino group, alkylenedioxy groups (e.g. C₁₋₃ alkylenedioxy groups such as methylenedioxy, ethylenedioxy, etc.), lower alkoxy groups (e.g. C₁₋₆ alkoxy groups such as methoxy, ethoxy, propoxy, isopropoxy, butoxy, isobutoxy, pentyloxy, hexyloxy, etc.), optionally halogenated lower alkoxy groups (e.g. mono-, di- or tri-halogeno-C₁₋₆ alkoxy groups such as chloromethyloxy, dichloromethyloxy, trichloromethyloxy, fluoromethyloxy, difluoromethyloxy, trifluoromethyloxy, 2-bromoethyloxy, 2,2,2-trifluoroethyloxy, pentafluorobutyloxy, 3,3,3-trifluoropropyloxy, 4,4,4-trifluorobutyloxy, 5,5,5-trifluoropentyloxy, 6,6,6-trifluorohexyloxy, etc), lower alkylthio groups (e.g. C₁₋₆ alkylthio groups such as methylthio, ethylthio, propylthio, isopropylthio, butylthio, isobutylthio, pentylthio, hexylthio, etc.), a carboxyl group, lower alkanoyl groups (e.g. formyl; C₁₋₆ alkyl-carbonyl groups such as acetyl, propionyl, butyryl, isobutyryl, etc.), lower alkanoyloxy groups (e.g. formyloxy; C₁₋₆ alkyl-carbonyloxy groups such as acetyloxy, propionyloxy, butyryloxy, isobutyryloxy etc.), lower alkoxycarbonyl groups (e.g. C₁₋₆ alkoxy-carbonyl groups such as methoxycarbonyl, ethoxycarbonyl, propoxycarbonyl, butoxycarbonyl, etc.), aralkyloxycarbonyl groups (e.g. C₇₋₁₁ aralkyloxy-carbonyl groups such as benzyloxycarbonyl, etc.), a thiocarbamoyl group, lower alkylsulfinyl groups (e.g. C₁₋₆ alkylsulfinyl groups such as methylsulfinyl, ethylsulfinyl, etc.), lower alkylsulfonyl groups (e.g. C₁₋₆ alkylsulfonyl groups such as methylsulfonyl, ethylsulfonyl, etc.), a sulfamoyl group, mono-lower alkylsulfamoyl groups (e.g. mono-C₁₋₆ alkylsulfamoyl groups such as methylsulfamoyl, ethylsulfamoyl, etc.), di-lower alkylsulfamoyl groups (e.g. di-C₁₋₆ alkylsulfamoyl groups such as dimethylsulfamoyl, diethylsulfamoyl, etc.), arylsulfamoyl groups (e.g. C₆₋₁₀ arylsulfamoyl groups such as phenylsulfamoyl, naphthylsulfamoyl, etc.), aryl groups (e.g. C₆₋₁₀ aryl groups such as phenyl, naphthyl, etc.), aryloxy groups (e.g. C₆₋₁₀ aryloxy groups such as phenyloxy, naphthyloxy, etc.), arylthio groups (e.g. C₆-₁₀ arylthio groups such as phenylthio, naphthylthio, etc.), arylsulfinyl groups (e.g. C₆₋₁₀ arylsulfinyl groups such as phenylsulfinyl, naphthylsulfinyl, etc.), arylsulfonyl groups (e.g. C₆₋₁₀ arylsulfonyl groups such as phenylsulfonyl, naphthylsulfonyl, etc.), arylcarbonyl groups (e.g. C₆₋₁₀ aryl-carbonyl groups such as benzoyl, naphthoyl, etc.), arylcarbonyloxy groups (e.g. C₆₋₁₀ aryl-carbonyloxy groups such as benzoyloxy, naphthoyloxy, etc.), optionally halogenated lower alkylcarbonylamino groups (e.g. C₁₋₆ alkyl-carbonylamino groups such as acetylamino, trifluoroacetylamino, etc.), optionally substituted carbamoyl groups (e.g. a group represented by the formula - CONR₂R₃ (wherein each R₂ and R₃ is a hydrogen atom, an optionally substituted hydrocarbon group or an optionally substituted heterocyclic group, further, R₂ and R₃ may form a ring together with the adjacent nitrogen atom)), optionally substituted amino groups (e.g. a group represented by the formula -NR₂R₃ (wherein R₂ and R₃ are as defined above, further, R₂ and R₃ may form a ring together with the adjacent nitrogen atom)), optionally substituted ureido groups(e.g. a group represented by the formula - NHCONR₂R₃ (wherein R₂ and R₃ are as defined above, further R₂ and R₃ may form a ring together with the adjacent nitrogen atom)), optionally substituted carboxamide groups (e.g. a group represented by the formula -NR₂COR₃ (wherein R₂ and R₃ are as defined above), optionally substituted sulfonamide groups (e.g. a group represented by the formula -NR₂SO₂R₃ (wherein R₂ and R₃ are as defined above), optionally substituted heterocyclic groups (as defined by one represented by R₂ and R₃) , etc.

Examples of the "hydrocarbon group" in the "optionally substituted hydrocarbon group" represented by R₂ and R₃ include lower alkyl groups (e.g. C₁₋₆ alkyl groups such as methyl, ethyl, propyl groups, etc.), lower alkenyl groups (e.g. C₂₋₆ alkenyl groups such as vinyl, allyl groups, etc.), lower alkynyl groups (e.g. C₂₋₆ alkynyl groups such as ethinyl, propargyl groups, etc.), cycloalkyl groups (e.g. C₃₋₈ cycloalkyl groups such as cyclopropyl, cyclobutyl, cyclopentyl, cyclohexyl groups, etc.), cycloalkenyl groups (e.g. C₃₋₈ cycloalkenyl groups such as cyclobutenyl, cyclopentenyl, cyclohexenyl groups, etc.), cycloalkylalkyl groups (e.g. C₃₋₈ cycloalkyl-C₁₋₆ alkyl groups such as cyclopropylmethyl, cyclobutylmethyl, cyclopentylmethyl, cyclohexylmethyl groups, etc.), cycloalkenylalkyl groups (e.g. C₃₋₈ cycloalkenyl-C₁₋₆ alkyl groups such as cyclobutenylmethyl, cyclopentenylmethyl, cyclohexenylmethyl groups, etc.), aryl groups (e.g. C₆₋₁₄ aryl groups such as phenyl, naphthyl groups, etc.), arylalkyl groups (e.g. C₆₋₁₄ aryl-C₁₋₆ alkyl groups such as benzyl, naphthylmethyl groups, etc.), and the like.

Examples of the "heterocyclic group" in the "optionally substituted heterocyclic group" represented by R₂ and R₃ include 5- to 12-membered monocyclic or condensed heterocyclic groups containing from 1 to 4 of one or two types of hetero atom(s) selected from a nitrogen atom, a sulfur atom and an oxygen atom, such as pyridyl, pyrrolidinyl, piperazinyl, piperidinyl, 2-oxoazepinyl, furyl, decahydroisoquinolyl, quinolinyl, indolyl, isoquinolyl, thienyl, imidazolyl, morpholinyl, etc. Example of the substituent in each of the "optionally substituted hydrocarbon group" and the "optionally substituted heterocyclic group" represented by R₂ and R₃ include halogen atoms (e.g. fluorine, chlorine, bromine, iodine, etc.), lower alkyl groups (e.g. C₁₋₆ alkyl groups such as methyl, ethyl, propyl groups, etc.), lower alkenyl groups (e.g. C₂₋₆ alkenyl groups such as vinyl, allyl groups, etc.), lower alkynyl groups (e.g. C₂₋₆ alkynyl groups such as ethinyl, propargyl groups, etc.), cycloalkyl groups (e.g. C₃₋₈ cycloalkyl groups such as cyclopropyl, cyclobutyl, cyclopentyl, cyclohexyl groups, etc.), lower alkoxy groups (e.g. C₁₋₆ alkoxy groups such as methoxy, ethoxy groups, etc.), a nitro group, a cyano group, a hydroxyl group, a thiol group, a carboxyl group, lower alkanoyl groups (e.g. formyl; C₁₋₆ alkyl-carbonyl groups such as acetyl, propionyl, butyryl groups, etc.), lower alkanoyloxy groups (e.g. formyloxy; C₁₋₆ alkyl-carbonyloxy groups such as acetyloxy, propionyloxy groups, etc.), lower alkoxycarbonyl groups (e.g. C₁₋₆ alkoxy-carbonyl groups such as methoxycarbonyl, ethoxycarbonyl, propoxycarbonyl groups, etc.), aralkyloxycarbonyl groups (e.g. C₇₋₁₇ aralkyloxy-carbonyl groups such as a benzyloxycarbonyl group, etc.), aryl groups (e.g. C₆₋₁₄ aryl groups such as phenyl, naphthyl groups, etc.), aryloxy groups (e.g. C₆₋₁₄ aryloxy groups such as phenyloxy, naphthyloxy groups, etc.), arylcarbonyl groups (e.g. C₆₋₁₄ aryl-carbonyl groups such as benzoyl, naphthoyl groups, etc.), arylcarbonyloxy groups (e.g.C₆₋₁₄ acryl-carbonyloxy groups such as benzoyloxy, naphthoyloxy groups, etc.), optionally substituted carbamoyl groups (e.g. carbamoyl; carbamoyl groups mono-substituted or di-substituted with C₁₋₆ alkyl groups such as methylcarbamoyl, dimethylcarbamoyl groups, etc.), optionally substituted amino groups (e.g. amino; amino groups mono-substituted or di-substituted with C₁₋₆ alkyl groups such as methylamino, dimethylamino, ethylamino, diethylamino, etc.), and the like. The number of substituents and the substitution positions are not particularly limited.

Examples of the ring formed by R₂ and R₃ together with the adjacent nitrogen atom include pyrrolidine, piperidine, homopiperidine, morpholine, piperazine, tetrahydroquinoline, tetrahydroisoquinoline, etc.

The "hydrocarbon group" in the "optionally substituted hydrocarbon group" represented by the above E, R, R₁ and G may have 1 to 5, preferably 1 to 3 substituent(s) as described above at possible position(s) of the hydrocarbon group. When the number of substituents is 2 or more, the respective substituents may be the same or different.

Examples of the "heterocyclic group" in the "optionally substituted heterocyclic group" represented by the above E, R and G include 5- to 12-membered aromatic heterocyclic groups and saturated or unsaturated non-aromatic heterocyclic groups containing 1 or more (preferably 1 to 4, more preferably 1 to 3) of one to three types (preferably one or two types) of hetero atom(s) selected from an oxygen atom, a sulfur atom and a nitrogen atom as an atom (ring atom) constituting the ring. Preferred examples of the "heterocyclic group" in the "optionally substituted heterocyclic group" represented by G include the above-described saturated oxygen-containing heterocyclic groups containing 1 to 4, more preferably 1 to 3 hetero atom(s) selected from an oxygen atom, a sulfur atom and a nitrogen atom as the ring atom, etc., and especially preferred examples include 5- to 12-membered saturated oxygen-containing heterocyclic groups, etc.

Examples of the "aromatic heterocyclic group" include aromatic monocyclic heterocyclic groups, aromatic condensed heterocyclic groups, etc.

Examples of the "aromatic monocyclic heterocyclic group" include 5- to 6-membered aromatic monocyclic heterocyclic groups such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridinyl, pyridazinyl, pyrimidinyl, pyrazinyl, triazinyl, etc.

Examples of the "aromatic condensed heterocyclic group" include 8- to 12-membered aromatic condensed heterocyclic groups such as benzofuranyl, isobenzofuranyl, benzothienyl, isobenzothienyl, indolyl, isoindolyl, 1H-indazolyl, benzimidazolyl, benzoxazolyl, 1,2-benzisoxazolyl, benzothiazolyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc. (preferably a heterocyclic ring formed by the above-described 5- or 6-membered aromatic monocyclic heterocyclic group condensed to a benzene ring or a heterocyclic ring formed by the same or different two 5- or 6-membered aromatic monocyclic heterocyclic groups condensed to each other).

Examples of the "saturated or unsaturated non-aromatic heterocyclic group" include 3- to 8-membered (preferably 5 to 6-membered) saturated or unsaturated (preferably saturated) non-aromatic heterocyclic group (aliphatic heterocyclic group) such as oxiranyl, azetidinyl, oxetanyl, thietanyl, pyrrolidinyl, tetrahydrofuryl, thioranyl, piperidinyl, tetrahydropyranyl, thianyl, morpholinyl, thiomorpholinyl, piperazinyl, azepanyl, oxepanyl, thiepanyl, oxazepanyl, thiazepanyl, azocanyl, oxocanyl, thiocanyl, oxazocanyl, thiazocanyl, etc. These may optionally be oxo-substituted, and examples thereof include 2-oxoazetidinyl, 2-oxopyrrolidinyl, 2-oxopiperidinyl, 2-oxoazepanyl, 2-oxoazocanyl, 2-oxotetrahydrofuryl, 2-oxotetrahydropyranyl, 2-oxothiolanyl, 2-oxothianyl, 2-oxopiperazinyl, 2-oxooxepanyl, 2-oxooxazepanyl, 2-oxothiepanyl, 2-oxothiazepanyl, 2-oxooxocanyl, 2-oxothiocanyl, 2-oxooxazocanyl, 2-oxothiazocanyl, etc. Preferred examples include 5-membered non-aromatic heterocyclic groups such as 2-oxopyrrolidinyl, etc.

Examples of the optional substituent of the "heterocyclic group" in the "optionally substituted heterocyclic group" represented by the above E, R and G include the same "substituents" as those in the "optionally substituted hydrocarbon group " represented by the above E, R, R₁ and G, etc.

The "heterocyclic group" in the "optionally substituted heterocyclic group" represented by E, R and G may have 1 to 5, preferably 1 to 3 the above substituent(s) at possible position(s) of the heterocyclic group. When the number of substituents is 2 or more, the respective substituents may be the same or different.

The case where R is bonded to W in the compound of the present invention will be described hereinafter. In the case where R is bonded to W, the position where R is bonded to W is not particularly limited as long as it is a possible position on each of R and W.

The possible position on R includes that on the "hydrocarbon group" and the "substituent" in the "optionally substituted hydrocarbon group" defined by the above R, and that on the "heterocyclic group" and the "substituent" in the "optionally substituted heterocyclic group" defined by the above R.

The possible position on W includes that on the "bivalent chain hydrocarbon group" in the "bivalent chain optionally substituted hydrocarbon group" defined by the above W, that on the "bivalent chain hydrocarbon group" defined by the above W₁ and W₂, that on the "hydrocarbon ring" in the "optionally substituted hydrocarbon ring" defined by the above ring Z, and that on the "heterocyclic ring" in the "optionally substituted heterocyclic ring" defined by the above ring Z.

R and W are bonded at a possible position to each other and form a ring together with the adjacent nitrogen atom. Examples of the ring include saturated nitrogen-containing rings (e.g. azetidine, pyrrolidine, piperidine, homopiperidine, etc.), unsaturated nitrogen-containing rings (e.g. tetrahydropyridine, etc.), aromatic nitrogen-containing rings (e.g. pyrrole, etc.), hetero rings containing 1 or more hetero atom(s) selected from the group consisting of nitrogen, oxygen and sulfur in addition to the nitrogen atom to which R and W are adjacent (e.g. piperazine, morpholine, etc.), condensed rings (e.g. indole, indoline, isoindole, isoindoline, tetrahydroquinoline, tetrahydroisoquinoline, etc.), and the like. Particularly preferred examples include 4- to 7-membered rings.

The ring formed by R and W bonded at a possible position to each other together with the adjacent nitrogen atom may have 1 to 4 substituent(s) at possible position(s). When the number of substituents is 2 or more, the respective substituents may be the same or different. Examples of the substituent include the same substituents as those for the "optionally substituted hydrocarbon group" and the "optionally substituted heterocyclic group" defined by R and the substituents for the "bivalent chain optionally substituted hydrocarbon group" defined by W. Specific examples of the substituent include halogen atoms (e.g. fluorine, chlorine, bromine, iodine, etc.), C₁₋₆ alkyl groups such as methyl, ethyl, propyl, isopropyl, butyl, isobutyl, sec-butyl, tert-butyl, pentyl, 1-ethylpropyl, hexyl, etc.), and the like.

Examples of the ring formed by bonding of R and W include 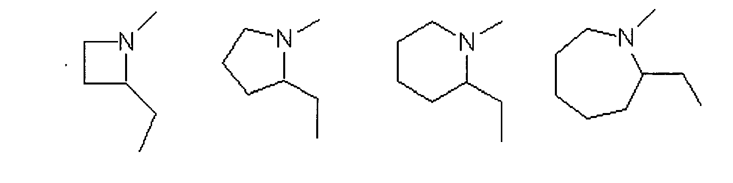 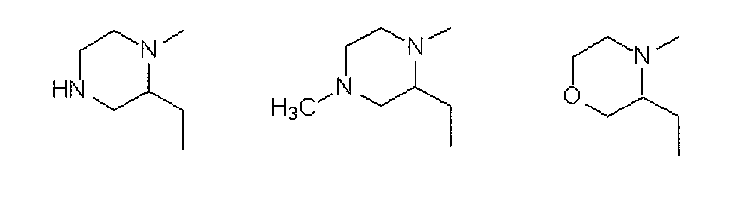 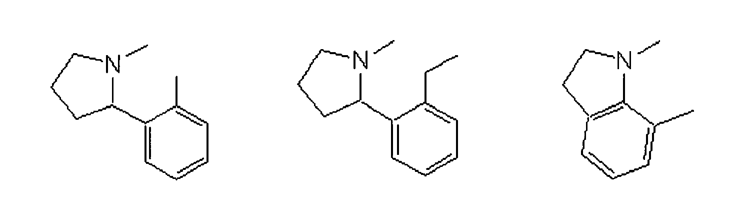 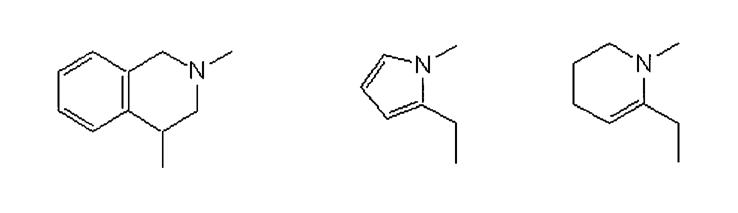 but the ring is not limited thereto. It should be understood by those skilled in the art that the above ring optionally have substituent(s) as defined above, and that their isomers are included.

X is a leaving group such as a halogen atom, a benzotriazolyl group, a (2,5-dioxypyrrolidin-1-yl)oxy group, etc. Inter alia, a halogen atom such as fluorine, chlorine, bromine, iodine, etc. is preferred, and chlorine is particularly preferred.

M is a hydrogen atom, a metal cation or a quaternary ammonium ion.

Examples of the "metal cation" in the present invention include alkali metal ions (e.g. Na⁺, K⁺, Li⁺, Cs⁺, etc.) and, inter alia, preferred is Na⁺.

Examples of the "quaternary ammonium ion" in the present invention include tetramethylammonium ion, tetraethylammonium ion, tetrapropylammonium ion, tetrabutylammonium ion, etc. and, inter alia, preferred is tetrabutylammonium ion.

In the compound (II), the acidic group and the inorganic base, the organic base or the like in the molecule may form a pharmaceutically acceptable basic salt, and the basic group and the inorganic acid, the organic acid or the like in the molecule may form a pharmaceutically acceptable acid addition salt.

Examples of the inorganic basic salt in the compound (II) include salts with alkali metals (e.g. sodium, potassium, etc.), alkaline earth metals (e.g. calcium, etc.), ammonia, etc., and examples of the organic basic salt in the compound (II) include salts with dimethylamine, triethylamine, piperazine, pyrrolidine, piperidine, 2-phenylethylamine, benzylamine, ethanolamine, diethanolamine, pyridine, collidine, etc.

Examples of the acid addition salt in the compound (II) include inorganic acid salts (e.g. hydrochloride, sulfate, hydrobromate, phosphate, etc.), organic acid salts (e.g. acetate, trifluoroacetate, succinate, maleate, fumarate, propionate, citrate, tartrate, lactate, oxalate, methanesulfonate, p-toluenesulfonate, etc.), and the like.

The compound (II) of the present invention includes a hydrate. Examples of the hydrate include hemihydrate to pentahydrate. Preferred is hemihydrate, monohydrate, 1.5 hydrate or dihydrate.

The compound (II) of the present invention includes racemates and optically active compounds. The optically active compound is preferably one with an enantiomeric excess (e.e.) of at lest 90%, more preferably one with an enantiomeric excess of at least 99%. The optically active isomer is preferably an R isomer represented by the formula 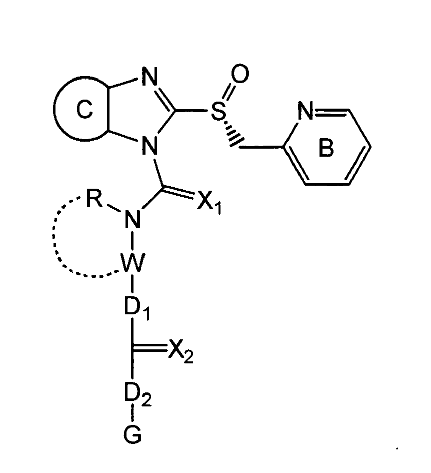 wherein the symbols are as defined above.

The compound (II) can be produced by known per se methods, for example, the method described in WO 03/105845.

The amount of the active ingredient to be formulated in the present invention varies depending on the kind of the active ingredient and dosage, and in the case of an enteric preparation containing PPI as the active ingredient, it is about 1% by weight to about 100% by weight, preferably about 5% by weight to about 50% by weight, based on the total powdered medicine, granules, fine granules, tablets, etc. (hereinafter they will generically be referred to as solid preparations) which are filled in the capsule preparation of the present invention. According to the present invention, a preparation containing an active ingredient at a high content can be prepared, and in the case of such a preparation containing the active ingredient at a high content, PPI may be contained in an amount of about 12% by weight to about 40% by weight, preferably about 12% by weight to about 30% by weight, more preferably about 14% by weight to about 30% by weight. When PPI is the benzimidazole type compound, in particular lansoprazole or an optically active isomer thereof, the amount can be as large as about 14% by weight to about 30% by weight.

Since PPI such as the imidazole type compound represented by the formula (I') has such properties that it is hardly soluble in water and is unstable to acids, an enteric coating is preferably applied to prepare an enteric preparation. An enteric coating does not dissolve in the stomach having low pH and containing a relatively larger amount of water, but dissolves in the small intestine having high pH and containing a smaller amount of water, whereby the imidazole type compound is dissolved and absorbed. That is, since a composition containing a benzimidazole type compound is required to be rapidly disintegrated in the small intestine, granules or fine granules which have a larger surface area, and which are more easily and rapidly disintegrated or dissolved are more desired. For easy administration, they are filled in a capsule to prepare a capsule preparation.

In the present invention, solid preparations such as granules, fine granules or tablets can be prepared by a known granulation method. In the case of granules and fine granules, examples of the granulation method include a rotary granulation method (e.g. centrifugal rotary granulation method), a fluidized bed granulation method, an agitation granulation method (e.g. rotary fluidized bed granulation method) and the like. Among them, a rotary granulation method and an agitation granulation method are preferred.

Specific examples of the rotary granulation method include CF apparatus manufactured by Freund Corporation, etc. Specific examples of the rotary fluidized bed granulation method include methods using SPIR-A-FLOW manufactured by Freund Corporation, Multiplex manufactured by POWREX Corporation, New-Marume manufactured by Fuji Paudal co., ltd and the like. A method for spraying a binder solution can be appropriately selected according to the kind of a granulator and, for example, it may be any of a top spraying method, a bottom spraying method, a tangential spraying method and the like.

In the case of the enteric preparations, preferably, the granules of the present invention have an active ingredient layer containing an active ingredient, an intermediate coating layer which is formed on the active ingredient layer, and an enteric coating layer or a controlled release coating layer which is formed on the intermediate coating layer.

As the solids preparations of the present invention such as granules, fine granules and tablets, preferably, the active ingredient layer is formed by coating core particles composed of one or more materials selected from sucrose, starch, lactose and microcrystalline cellulose with the benzimidazole type compound for obtaining granules having higher sphericity and narrower particle size distribution. For example, the granules or fine granules having core particles can be prepared by the method described in JP 63-301816 A. That is, the granules can be obtained by a method wherein a sugar core is coated with a dusting powder containing the benzimidazole type compound having antiulcer activity, a basic metal salt, an excipient, a disintegrating agent and the like, while a binder solution such as hydroxypropylcellulose are spraying on the sugar cores. Examples of the core particles include Nonpareil obtained by coating sucrose (75 parts by weight) with corn starch (25 parts by weight) by a known per se method, spherical core granules using crystalline cellulose, etc. Alternatively, the core particles per se may be an active ingredient which becomes the above active ingredient. An average particle size of the core particles is generally 14 to 80 mesh.

Examples of the cores include a spherical granulated material of sucrose and starch, a spherical granulated material of crystalline cellulose, a spherical granulated material of crystalline cellulose and lactose, etc.

It is desirable that cores are as uniformly spherical as possible so as to reduce variation of coating.

The proportion of the coating layer to the cores can be selected from within such a range that the dissolution property of the benzimidazole type compound and a particle size of the granules can be controlled. For example, the proportion is usually about 0.2 part by weight to about 5 parts by weight, preferably about 0.1 part by weight to about 5 parts by weight based on 1 part by weight of the cores.

The coating layer with which the active ingredient layer is coated may be formed of multi layers. The multi coating layers may contain various coating layers such as a controlled release coating layer and a coating layer for subcoating in addition to an intermediate coating layer containing no medicine and an enteric coating layer, and a particular combination of those coating layers may be appropriately selected.

In solid preparations such as enteric coating granules, fine granules and tablets containing an unstable active ingredient such as the imidazole type compound, etc., it is preferable to arrange an intermediate coating layer between the main ingredient layer containing the imidazole type compound, etc. and an enteric coating layer to block direct contact between the layers because the enteric coating layer is an acidic substance. Further, also in a case where a controlled release coating layer is formed, it is preferable to preliminarily arrange an intermediate coating layer considering instability of the amorphous compound.

Such an intermediate coating layer can be a coating layer which can prevent contact between the imidazole type compound as the active ingredient and an enteric coating layer, and the amount and material of the coating layer are not limited as long as this purpose can be achieved. For example, there is a layer in which a saccharide such as sucrose (refined white sugar (pulverized (powdered sugar) or not pulverized), etc.), starch sugar such as corn starch, lactose, honey, sugar alcohol (D-mannitol, erythritol, etc.), etc. is appropriately formulated into a polymer base such as low-substituted hydroxypropylcellulose, hydroxypropylcellulose, hydroxypropylmethylcellulose (e.g. TC-5, etc.), polyvinylpyrrolidone, polyvinyl alcohol, methylcellulose, hydroxyethylmethylcellulose and the like. In addition, to the intermediate coating layer can be appropriately added an excipient (e.g. masking agent (titanium oxide, etc.), and an antistatic agent (titanium oxide, talc, etc.)) which are added for formulating into a preparation as needed and as described hereinafter.

The amount of the intermediate coating layer to be coated is usually about 0.02 part by weight to about 1.5 parts by weight, preferably about 0.05 part by weight to about 1 part by weight based on 1 part by weight of the solid preparation such as granules, fine granules or tablets containing, for example, the imidazole type compound. Coating can be performed according to a conventional method. For example, preferably, these intermediate coating layer ingredients are diluted with purified water or the like to obtain a liquid mixture, followed by spraying it for coating.

The granules, the fine granules or the tablets of the present invention may be coated with an "enteric coating layer". Such an "enteric coating layer" is dissolved at pH 5.5 or higher and starts to release the medicine. Examples of a material forming such an enteric coating layer include aqueous enteric polymer bases such as cellulose acetate phthalate (CAP), hydroxypropylmethylcellulose phthalate, hydroxymethylcellulose acetate succinate, methacrylic acid copolymer, carboxymethylethylcellulose, shellac and the like, sustained-release bases such as ethyl acrylate-methacrylic acid copolymer and the like, and plasticizers such as water-soluble polymer, triethyl citrate, polyethylene glycol, acetylated monoglyceride, triacetin, castor oil and the like. They can be used by mixing one or more thereof.

The enteric coating layer is an enteric polymer base, preferably an enteric methacrylic acid copolymer.

The amount of the enteric coating layer to be coated is about 10% by weight to about 70% by weight, preferably about 10% by weight to about 50% by weight, more preferably about 15% by weight to about 40% by weight based on the total amount of the granules before coating of the enteric coating.

The solid preparation in the present invention, particularly the granules, the fine granules or the tablets, may be a solid preparation from which the release of the medicine is controlled by arranging a controlled release coating layer. For example, it may be a modified preparation so that the medicinal effect will be effectively achieved by forming a "time-release type controlled release coating layer". Such a "time-release type controlled release coating layer" includes a system which is a combination of a controlled coating and a coating having disintegration function, and in which, while the controlled coating per se does not dissolve, it absorbs moisture through pores formed on the coating, and after a lapse of an aimed certain period of time, the controlled coating is disintegrated together with the coating having disintegration function, whereby the active ingredient will be quickly released.

The solid preparation to be used in the present invention may also be a preparation having a prolonged medicinal effect by forming a "pH-dependent soluble or diffusion-controlled type controlled release coating layer". Namely, the coating layer includes a coating which will be dissolved in a pH region which is different from that for an ordinary enteric coating (e.g. pH 6 or higher, preferably pH 6.5 or higher, usually from higher or equal to pH 6 to lower or equal to 7.5), i.e. which will be pH-dependently dissolved and disintegrated to release the medicine (this coating is generally considered to be one of enteric coatings, but herein, it is considered as a pH-dependent soluble controlled release coating) and a diffusion-controlled type controlled release coating in which, while the coating per se does not dissolve, it controls the release of an active ingredient through pores formed on the coating. Herein, "pH-dependent" means that the active ingredient is released in an environment at certain pH or higher. The pH means that adjusted usually with Mcllvaine buffer solution or Clark-Lubs buffer solution. The pH of the film to be pH-dependently dissolved means this pH throughout the specification.

Examples of a substance for a controlled-release coating to pH-dependently control the release of a pharmaceutically active ingredient include hydroxypropylmethylcellulose phthalate (HP-55, HP-50 manufactured by Shin-Etsu Chemical Co., Ltd.), cellulose acetate phthalate, carboxymethylethylcellulose (CMEC manufactured by Freund Corporation), methacrylic acid-methylmethacrylate copolymer (EUDRAGIT L100, manufactured by Rohm), methacrylic acid-ethyl acrylate copolymer (EUDRAGIT L100-55, EUDRAGIT L30D-55, manufactured by Rohm), hydroxypropylcellulose acetate succinate (HPMCAS, manufactured by Shin-Etsu Chemical Co., Ltd.), polyvinyl acetate phthalate, shellac, etc. They may be used alone or in combination of two or more polymers, or in combination of two or more polymers successively coated. It is desirable to use coating substances alone or in combination as the case requires so that the coating will be dissolved at preferably pH of 6.0 or higher, more preferably pH of 6.5 or higher, furthermore preferably pH of 6.75 or higher. The upper limit of the pH is not particularly determined but usually preferred is a polymer which will be dissolved at a pH of 7.5 or lower. Further, for coating, it is possible to use a plasticizer, a stabilizer, etc. including polyethylene glycol, dibutyl sebacate, diethyl phthalate, triacetin, triethyl citrate, etc. as the case requires. The amount of the coating substance is preferably 5% to 100% based on the core particles.

Further, a diffusion-controlled type controlled release film which controls the release of an active ingredient by diffusion can be produced by coating granules with a mixture of aminoalky-methacrylate copolymer (EUDRAGIT RS, RL, manufactured by Rohm), ethyl acrylate-methyl methacrylate copolymer (EUDRAGIT NE 30D, manufactured by Rohm), ethyl cellulose or the like and a hydrophilic pore-forming substance such as HPMC, HPC, carboxyvinyl polymer, polyethylene glycol 6000, lactose, mannitol, an organic acid in a certain proportion.

The solid preparation of the present invention may be one having any of a powdered medicine, fine granules, granules and tablets filled in a capsule.

In the capsule preparation of the present invention, various solid preparations such as a powdered medicine, granules, fine granules and tablets in combination may be filled in a capsule. Especially, it is possible to contain two or more solid preparations including granules, fine granules and tablets in combination. Solid preparations such as granules, fine granules and tablets containing different active ingredients may be combined. Further, solid preparations such as granules, fine granules and tablets having different release properties may be combined. As one form of such solid preparations having different release properties, a controlled release solid preparation may be prepared by arranging the above-described controlled release coating layer. Particularly, in the case of a capsule preparation containing as an active ingredient the imidazole type PPI, an optically active isomer thereof or a salt thereof, by formulating a capsule preparation containing fine granules, granules or tablets having an enteric coating layer and fine granules, granules or tablets having a controlled release coating layer in combination to prepare the capsule preparation, the medicinal effect can be quickly achieved after the administration and, in addition, the medicinal effect can be maintained by the controlled release solid preparation, and medicinal effect peaks can appear plural times as the case requires

The capsule can be filled with the solid preparation including a powdered medicine in accordance with a know method.

Further, additives can be used, and examples thereof include excipients for formulating into pharmaceutical preparations (e.g. glucose, fructose, lactose, sucrose, D-mannitol, erythritol, maltitol, trehalose, sorbitol, corn starch, potato starch, wheat starch, rice starch, crystalline cellulose, anhydrous silicic acid, anhydrous calcium phosphate, precipitated calcium carbonate, calcium silicate, etc.), binders (e.g. hydroxypropylcellulose, hydroxypropylmethylcellulose, polyvinylpyrrolidone, methylcellulose, polyvinyl alcohol, sodium carboxymethylcellulose, partial alpha-starch, alpha-starch, sodium alginate, pullulan, gum arabic powder, gelatin etc.), disintegrating agents (e.g. low-substituted hydroxypropylcellulose, carmellose, calcium carmellose, sodium carboxymethylstarch, sodium crosscarmellose, crosspovidone, hydroxypropylstarch, etc.), corrigents (e.g. citric acid, ascorbic acid, tartaric acid, malic acid, aspartame, potassium acesulfame, sormatin, saccharin sodium, dipotassium glycyrrhizinate, sodium glutamate, sodium 5'-inosinate, sodium 5'-guanylate, etc.), surfactants (e.g. polysorbates (polysorbate 80, etc.), polyoxyethylene-polyoxypropylene copolymer, sodium laurylsulfate, etc.), plasticizers (e.g. polyethylene glycol, polypropylene glycol, triethyl citrate, etc.), flavors (e.g. lemon oil, orange oil, menthol, mint oil, etc.), lubricants (e.g. magnesium stearate, sucrose fatty acid esters, sodium stearyl fumarate, stearic acid, talc, polyethylene glycol, etc.), colorants (e.g. titanium oxide, edible Yellow No. 5, edible Blue No. 2, iron sesquioxide, yellow iron sesquioxide etc.), antioxidants (e.g. sodium ascorbate, L-cysteine, sodium sulfite, etc.), masking agents (e.g. titanium oxide, etc.), antistatic agents (e.g. talc, light anhydrous silicic acid, titanium oxide, etc.) and the like.

The particle size of raw materials used for these materials is not particularly limited, but particles of about 500 µm or smaller is preferred from a viewpoint of manufacturing properties and administration properties.

In a case where the imidazole type PPI such as the compound (I') are used as the pharmaceutically active ingredient in the capsule preparation of the present invention, since they have excellent antiulcer activity, gastric acid secretion inhibiting activity, mucosa protecting activity, anti-Helicobacter pylori activity and the like, and has low toxicity, they are useful for medicine. In this case, the capsule preparation of the present invention can be administered to a mammal (e.g. human, monkey, sheep, horse, dog, cat, rabbit, rat, mouse, etc.) orally for the purpose of treating and preventing peptic ulcers (e.g. gastric ulcer, duodenal ulcer, stomal ulcer, etc.), Zollinger-Ellison syndromes, gastritis, reflux esophagitis, symptomatic gastroesophageal reflux disease (symptomatic GERD)) without esophagitis, NUD (non ulcer dyspepsia), stomach cancer (including stomach cancer accompanied with promotion of production of interleukin-β due to genetic polymorphism of interleukin-1), gastric MALT lymphoma and the like, eradicating Helicobacter pylori, suppressing upper gastrointestinal hemorrhage due to peptic ulcers, acute stress ulcer and hemorrhagic gastritis, suppressing upper digestive tract hemorrhagic due to invasion stress (stress resulting from major operation requiring postoperative intensive management, and cerebrovascular disorder, head trauma, multiple organ failure and diffuse burn requiring intensive care), treating and preventing ulcers resulting from nonsteroidal antiinflammatory medicaments; treating and preventing excess stomach acid and ulcer due to post-operation stress, and the like. For eradicating Helicobacter pylori, etc., the capsule preparation of the present invention may be used together with other active ingredient(s) (e.g. 1 to 3 active ingredient(s)).

Examples of the "other active ingredient(s)" include anti-Helicobacter pylori active substances, antimicrobial agents such as imidazole type compounds, quinolone type compounds, and bismuth salts. Especially, medicines comprising a combination of the granules or the capsule preparation of the present invention and an antimicrobial agent are preferred. Among them, a combination with anti-Helicobacter pylori active substances, or antimicrobial agents such as imidazole type compounds is preferred. Examples of the "anti-Helicobacter pylori active substance" include penicillin antibiotics (e.g. amoxicillin, benzylpenicillin, piperacillin, mecillinam, etc.), cephem antibiotics (e.g. cefixime, cefaclor, etc.), macrolide antibiotics (e.g. erythromycin antibiotics such as erythromycin, clarithromycin, etc.), tetracycline antibiotics (e.g. tetracycline, minocycline, streptomycin, etc.), aminoglycoside antibiotics (e.g. gentamycin, amikacin, etc.), imipenem and the like. Among them, penicillin antibiotics and macrolide antibiotics are preferred.

Examples of the "imidazole type compound" include metronidazole, miconazole, etc. Examples of the "bismuth salt" include bismuth acetate, bismuth citrate, etc. Antibacterial agents of "quinolone type compounds" are also preferred, and examples thereof include ofloxacin, ciproxacin, etc. Especially, for eradicating Helicobacter pylori, it is preferred to use the granules or the capsule preparation of the present invention in combination with penicillin antibiotics (e.g. amoxicillin, etc.) and/or erythromycin antibiotics (e.g. clarithromycin etc.).

A daily dosage varies depending on the degree of symptom, the age, sex and weight of subject, the administration time, interval, and the kind of the active ingredient, etc., and is not specifically limited. For example, when the imidazole type compound such as the compound (I') is orally administered to an adult (60 kg) as an antiulcer agent, a dosage is about 0.5 to 1500 mg/day, preferably about 5 to 150 mg/day in terms of an active ingredient. These imidazole type compound-containing preparations may be administered once or by dividing into 2 to 3 times daily.

Further, it is possible to stabilize by a package form so as to improve stability of the solid preparation of the present invention during storage, transportation, etc. For example, by using a package form such as a packaging having moisture permeability suppressed or a packaging having a desiccating agent included therein, it is possible to improve stability of the capsule preparation containing the imidazole type compound of the present invention. By such a package form, moisture to which the capsule preparation is in direct contact will be reduced and the stability will improve.

Hereinafter, the present invention will be illustrated in more detail by Examples, but the present invention is not limited by them.

In the following Examples, as hydroxypropylcellulose (HPC-L), sterile talc, hydroxypropylmethylcellulose, polyethylene glycol 6000 and titanium oxide, products which complied with the Japanese Pharmacopoeia, Fourteenth Edition were used.

### Example 1

The composition is shown in Table 1. Lansoprazole R-isomer, magnesium carbonate, sucrose (pulverized sucrose) and low-substituted hydroxypropylcellulose are thoroughly mixed to obtain a main ingredient mixture. Spherical granules consisting of sucrose and starch are placed in a centrifugal rotary granulator (CF apparatus, manufactured by Freund Corporation), and the above main ingredient mixture is coated while spraying a hydroxypropylcellulose solution (2%: W/W) to obtain spherical granules. The coating operation conditions are as follows: rotor rotating speed: 240 rpm, spray rate: 20 g/min, spray air pressure: 0.1 kg/cm², and slit air pressure: 0.1 kg/cm ² The resultant spherical granules are dried in a vacuum at 40°C for 16 hours and passed through a round sieve to obtain granules of 710 µm to 1400 µm.

The above active ingredient granules are coated with a coating liquid for an intermediate layer using a fluidized granulation coating machine (MP-10, manufactured by POWREX CORPORATION). The coating operation conditions are as follows: inlet air volume: 1.5 m³/min, inlet air temperature: 65°C, spray rate: 12 g/min, and spray air pressure: 3 kg/cm². The resultant spherical granules are dried in a vacuum at 40°C for 16 hours and passed through a round sieve to obtain intermediate layered granules of 710 µm to 1400 µm.

The above intermediate layered granules are coated with a coating liquid for enteric coating using a fluidized granulation coating machine (MP-10, manufactured by POWREX CORPORATION). The coating operation conditions are as follows: inlet air volume: 1.5 m³/min, inlet air temperature: 65°C, spray rate: 15 g/min, and spray air pressure: 3 kg/cm². The resultant spherical granules are dried in a vacuum at 40°C for 16 hours and passed through a round sieve to obtain enteric granules of 850 µm to 1400 µm.

The above intermediate layered granules are coated with a coating liquid for pH-dependent soluble controlled release coating using a fluidized granulation coating machine (MP-10, manufactured by POWREX CORPORATION). The coating operation conditions are as follows: inlet air volume: 1.5 m³/min, inlet air temperature: 45°C, spray rate: 15 g/min, and spray air pressure: 3 kg/cm². The resultant spherical granules are dried in a vacuum at 40°C for 16 hours and passed through a round sieve to obtain pH-dependent soluble controlled release granules of 1000 µm to 1700 µm.

Each of the resultant enteric granules and pH-dependent soluble controlled release granules are mixed with talc and light anhydrous silicic acid. Both of 87 mg (corresponding to 22.5 mg of Lansoprazole R-isomer) of the resultant enteric mixed granules and 315 mg (corresponding to 67.5 mg of Lansoprazole R-isomer) of the resultant pH-dependent soluble controlled release mixed granules are filled into a No. 1 pullulan capsule.

Further, both of 87 mg (corresponding to 22.5 mg of Lansoprazole R-isomer) of the resultant enteric mixed granules and 315 mg (corresponding to 67.5 mg of Lansoprazole R-isomer) of the resultant pH-dependent soluble controlled release mixed granules are filled into a No. 1 PEG-containing gelatin capsule.

**Table 1**

| COMPOSITION | | |
|---|---|---|
| <Composition of active ingredient granules> | | |
| | Enteric mixed granules | pH-dependent soluble controlled release mixed granules |
| Spherical granules | 15.0 mg | 45.0 mg |
| consisting of sucrose and starch | | |
| (Spreading mixture of main ingredient) | | |
| Lansoprazole R-isomer | 22.5 mg | 67.5 mg |
| Magnesium carbonate | 6.0 mg | 18.0 mg |
| Sucrose (pulverized sucrose) | 14.82 mg | 44.46 mg |
| Low-substituted | 4.5 mg | 13.5 mg |
| hydroxypropylcellulose (Binder solution) | | |
| Hydroxypropylcellulose | 0.18 mg | 0.54 mg |
| Purified water | 8.82 mg | 26.46 mg |
| Total (solid content) | 63.0 mg | 189.0 mg |

| <Composition of coating solution for intermediate layer> | | |
|---|---|---|
| | Enteric mixed granules | pH-dependent soluble controlled release mixed granules |
| Hydroxypropyl-methylcellulose | 3.94 mg | 11.82 mg |
| Talc | 1.58 mg | 4.74 mg |
| Titanium oxide | 2.36 mg | 7.08 mg |
| Purified water | 70.92 mg | 212.76 mg |
| Total (solid content) | 7.86 mg | 23.58 mg |

| <Composition of intermediate layered granules> | | |
|---|---|---|
| | Enteric mixed granules | pH-dependent soluble controlled release mixed granules |
| main ingredient granules | 63.0 mg | 189.0 mg |
| Coating solution for intermediate layer | 7.86 mg | 23.58 mg |
| Total | 70.88 mg | 212.64 mg |

| <Composition of coating solution for enteric coating> | | |
|---|---|---|
| | Enteric mixed granules | pH-dependent soluble controlled release mixed granules |
| Methacrylic acid copolymer (1) | 35.1 mg (solid component 10.53 mg) | - |
| Polyethylene glycol 6000 | 1.05 mg | - |
| Polysorbate 80 | 0.48 mg | - |
| Titanium oxide | 1.05 mg | - |
| Talc | 2.92 mg | - |
| Purified water | 48.46 mg | - |
| Total (solid content) | 16.03 mg | - |

| <Composition of enteric granules> | | |
|---|---|---|
| | Enteric mixed granules | pH-dependent soluble controlled release mixed granules |
| Intermediate layered granules | 70.88 mg | - |
| Coating liquid for enteric coating | 16.03 mg | - |
| Total | 86.91 mg | - |
| <Composition of coating solution for pH-dependent soluble controlled release coating> | | |

| | Enteric mixed granules | pH-dependent soluble controlled release mixed granules |
|---|---|---|
| Methacrylic acid copolymer (2) | - | 47.85 mg |
| Methacrylic acid copolymer (3) | - | 15.96 mg |
| Triethyl citrate | - | 6.36 mg |
| Talc | - | 31.89 mg |
| Ethanol | - | 826.69 mg |
| Purified water | - | 91.85 mg |
| Total (solid content) | - | 102.06 mg |

| <Composition of pH-dependent soluble controlled release granules> | | |
|---|---|---|
| | Enteric mixed granules | pH-dependent soluble controlled release mixed granules |
| Intermediate layered granules | - | 212.64 mg |
| Coating solution for pH-dependent soluble controlled release coating | - | 102.06 mg |
| Total | - | 314.7 mg |

| <Composition of enteric mixed granules and pH-dependent soluble controlled release mixed granules> | | |
|---|---|---|
| | Enteric mixed granules | pH-dependent soluble controlled release mixed granules |
| Enteric granules | 86.91 mg | - |
| pH-dependent soluble controlled release granules | - | 314.7 mg |
| Talc | 0.045 mg | 0.195 mg |
| Light anhydrous silicic acid | 0.045 mg | 0.195 mg |
| Total | 87.0 mg | 315.0 mg |

| <Composition of capsule preparation (corresponding to 90 mg of Lansoprazole R-isomer)> | | |
|---|---|---|
| | Pullulan | PEG-containing |

| | capsule preparation | gelatin capsule preparation |
|---|---|---|
| Enteric mixed granules | 87.0 mg | 87.0 mg |
| pH-dependent soluble controlled release mixed granules | 315.0 mg | 315.0 mg |
| Pullulan capsule | No. 1 capsule | - |
| PEG-containing capsule | - | No. 1 capsule |

### Example 2

185 mg of the pH-dependent soluble controlled release granules prepared in Example 1 were filled into a No. 1 pullulan capsule.

### Comparative Example 1

185 mg of the pH-dependent soluble controlled release granules prepared in Example 1 were filled into a No. 1 gelatin capsule.

### Experiment Example 1

The capsule preparations prepared in Example 2 and Comparative Example 1 were stored at 25°C at 11%RH for two weeks in equilibrium. The capsule preparations after storage were horizontally held, and the fracture ratio of the capsules was evaluated by pressurizing with an autograph (5,000 kgf load cell) (rate of compression: 300 mm/min, 60% deformation ratio (40% displacement), n=10). As a result, the fracture ratio of the gelatin capsules was 70% (7 capsules among 10 fractured), whereas the fracture ratio of the pullulan capsules was 30% (3 capsules among 10 fractured), which indicated that the pullulan capsules are stable in a low moisture state (11%ERH) as compared with the gelatin capsules.

### Industrial Applicability

According to the present invention, a stable capsule preparation containing a medicine unstable to moisture can be provided by using a capsule stable in a low moisture state. The capsule preparation of the present invention comprising, for example, PPI of an imidazole type compound as the main ingredient is useful as a preparation for treating or preventing peptic ulcers, gastritis, reflux esophagitis and symptomatic gastroesophageal reflux disease (symptomatic GERD) or a preparation for eradicating Helicobacter pylori.

## Claims

1. A capsule preparation, which comprises a medicine unstable to moisture, is stable in a low moisture state and has pH-independent disintegration properties.

2. The capsule preparation according to claim 1, which is stable in a low moisture state which is less or equal to relative humidity of about 35%.

3. The capsule preparation according to claim 1, wherein the main component of the capsule is a gelatin containing polyethylene glycol.

4. The capsule preparation according to claim 1, wherein the main component of the capsule is a water-soluble polysaccharide.

5. The capsule preparation according to claim 1, wherein the main component of the capsule is pullulan.

6. The capsule preparation according to claim 1, which combines a capsule shell comprising gelatin containing polyethylene glycol as the main component and a capsule shell comprising pullulan as the main component.

7. The capsule preparation according to claim 1, wherein the medicine unstable to moisture is a proton pump inhibitor (PPI).

8. The capsule preparation according to claim 7, wherein the PPI is an imidazole type compound represented by the formula (I'): 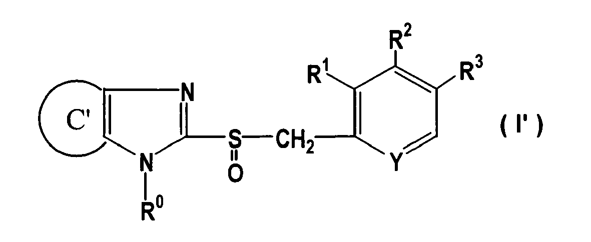 wherein the ring C' is an optionally substituted benzene ring or an optionally substituted aromatic mono-heterocyclic ring, R° is a hydrogen atom, an optionally substituted aralkyl group, an acyl group or an acyloxy group, each of R¹, R² and R³ which may be the same or different, and is a hydrogen atom, an optionally substituted alkyl group, an optionally substituted alkoxyl group, or an optionally substituted amino group, and Y is a nitrogen atom or CH, or an optically active isomer thereof or a salt thereof.

9. The capsule preparation according to claim 8, wherein C' is an optionally substituted benzene ring.

10. The capsule preparation according to claim 7, wherein the PPI is lansoprazole, omeprazole, rabeprazole, pantoprazole, tenatoprazole, or an optically active isomer thereof or a salt thereof.

11. The capsule preparation according to claim 7, wherein the PPI is lansoprazole.

12. The capsule preparation according to claim 7, wherein the PPI is an optically active isomer (R-isomer) of lansoprazole.

13. The capsule preparation according to claim 1, wherein the medicine unstable to moisture is a prodrug of PPI.

14. The capsule preparation according to claim 1, wherein the content in the capsule is a powdered medicine.

15. The capsule preparation according to claim 1, wherein the content in the capsule is fine granules optionally coated, granules optionally coated and/or tablets optionally coated.

16. The capsule preparation according to claim 15, which contains at least two solid preparations selected from fine granules, granules and tablets in combination.

17. The capsule preparation according to claim 16, wherein the combined solid preparations have different medicine release properties.

18. The capsule preparation according to claim 16, wherein at least one of the combined solid preparations has a coating layer.

19. The capsule preparation according to claim 18, wherein the coating layer is an enteric coating layer.

20. The capsule preparation according to claim 18, wherein the coating layer contains a controlled-release coating layer.

21. The capsule preparation according to claim 20, wherein the controlled-release coating layer is a pH-dependent soluble controlled-release coating film containing a polymer soluble within a range of pH 6.0 to pH 7.5.

22. The capsule preparation according to claim 21, wherein the controlled-release coating layer is a diffusion-control type controlled-release film.

23. The capsule preparation according to claim 21, wherein the controlled-release coating layer is a time release type controlled-release coating film.

24. The capsule preparation according to claim 16, which contains fine granules, granules or tablets having an enteric coating layer in combination with fine granules, granules or tablets having a controlled-release coating layer.
